(19) Europäisches Patentamt
European Patent Office
Office européen des brevets

(11) EP 1 734 046 B1

(12) **EUROPEAN PATENT SPECIFICATION**

(45) Date of publication and mention of the grant of the patent:
**01.09.2010 Bulletin 2010/35**

(21) Application number: **05765113.5**

(22) Date of filing: **24.06.2005**

(51) Int Cl.:
***C07H 15/04*** *(2006.01)* ***A61K 31/7032*** *(2006.01)*
***A61P 35/00*** *(2006.01)* ***A61P 35/02*** *(2006.01)*
***A61P 43/00*** *(2006.01)*

(86) International application number:
**PCT/JP2005/011630**

(87) International publication number:
**WO 2006/001374 (05.01.2006 Gazette 2006/01)**

(54) **Sulfopyranosylacylglycerol derivatives for use in combination with irradiation in an anti-tumor radiation treatment**

Sulfopyranosylacylglycerol-Derivate zur Anwendung in Kombination mit Bestrahlung in einer Tumor-Radiotherapie

Dérivés de sulfopyranosylacyglycerol pour la mise en oeuvre en combinaison avec de l'irradiation dans un traitement de tumeur par radiothérapie

(84) Designated Contracting States:
**AT BE BG CH CY CZ DE DK EE ES FI FR GB GR HU IE IS IT LI LT LU MC NL PL PT RO SE SI SK TR**

(30) Priority: **24.06.2004 JP 2004186480**
**24.12.2004 JP 2004374445**

(43) Date of publication of application:
**20.12.2006 Bulletin 2006/51**

(73) Proprietor: **Toyo Suisan Kaisha, Ltd.**
**Tokyo 108-8501 (JP)**

(72) Inventors:
- **SAKIMOTO, Ippei**
  **Toyo Suisan Kaisha Ltd.**
  **Tokyo, 108-8501 (JP)**
- **MIURA, Masahiko**
  **1-5-45 Hushima, Bunkyo-ku,**
  **Tokyo 113-8549 (JP)**
- **KATAOKA, Keiko**
  **Tokyo Medical and Dental University**
  **Tokyo (JP)**
- **SAKAGUCHI, Kengo**
  **Tokyo University of Science**
  **Ciba-ken (JP)**
- **SUGAWARA, Fumio**
  **Tokyo University of Science**
  **Ciba-ken (JP)**
- **OHTA, Keisuke**
  **Toyo Suisan Kaisha Ltd.**
  **Tokyo, 108-8501 (JP)**
- **YAMAZAKI, Takayuki**
  **Toyo Suisan Kaisha Ltd.**
  **Tokyo, 108-8501 (JP)**

(74) Representative: **Jennings, Nigel Robin et al**
**Kilburn & Strode LLP**
**20 Red Lion Street**
**London WC1R 4PJ (GB)**

(56) References cited:
**WO-A-96/34872 WO-A1-00/51622**
**WO-A1-00/52020 WO-A1-00/52021**
**JP-A- 57 035 516 JP-A- 57 067 518**
**JP-A- 2000 143 516 JP-A- 2000 212 087**

- **OHTA K. ET AL: 'Action of a New Mammalian DNA Polymerase Inhibitor, Sulfoquinovosyl diacylglycerol' BIOLOGICAL & PHARMACEUTICAL BULLETIN vol. 22, no. 2, 1999, pages 111 - 116, XP002926007**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 734 046 B1

## Description

### Technical Field

The present invention relates to a radiosensitizer containing, as an active ingredient, sulfopyranosylacylglycerol represented by the general formula (1) or a pharmaceutically acceptable salt thereof for use in combination with irradiation in an anti-tumor radiation treating method.

### Background Art

Currently, in Japan, malignant tumor, cardiac disease and cerebrovascular disease account for about 60% of death cause. Among them, malignant tumor has the top ranking of death cause, and tends to increase. As therapy for malignant tumor, operation therapy, chemotherapy and radiation therapy are known as the three major therapies. In recent years, quality of life (QOL) of a patient is emphasized, and much attention is being paid to radiation therapy.

Currently, clinically applicable radiosensitizers consisting of a chemical or a pharmaceutical substance, which enhance the therapeutic effect when administered with radiation in radiation therapy, include halogenated pyrimidine and a hypoxic cell radiosensitizer (e.g., see Radiobiology for the Radiologist (Fourth Edition), Eric J. Hall et al., J.B. Lippincott Company ("Radiobiology for the Radiologist", translated by Muneyasu Urano, Shinohara shinsha Inc.)). As the halogenated pyrimidine, 5-iododeoxyuridine and the like are known. In addition, as the hypoxic cell radiosensitizer, misonidazol and the like are known. However, these known radiosensitizers have problems to be solved, such as gastrointestinal disorder, peripheral neurotoxicity and a problem of other side effects, and have scarcely been put into practice.

Ohta et al., Biol. Pharm. Bull. 22(2) 111-116 (1999) discloses that a sulfoquinovosyldiacylglycerol compound represented by the following formula could synergistically enhance the cytocidal effect of an anti-cancer chemotherapy agent

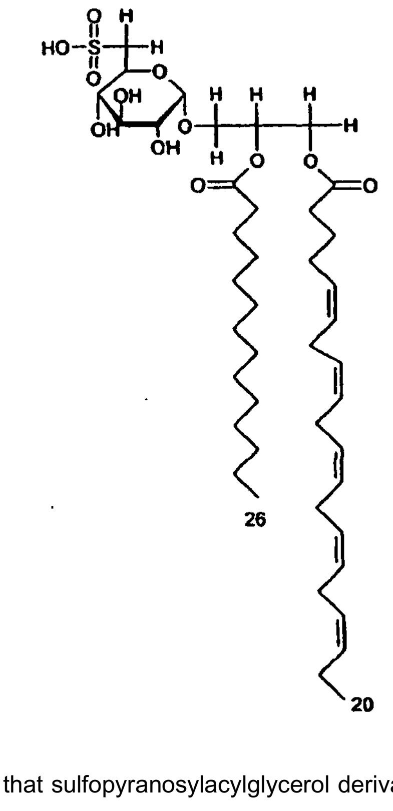

WO 00/51622 A1 discloses that sulfopyranosylacylglycerol derivatives represented by the following formula have anti-cancer activity

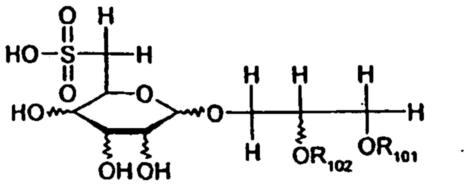

WO 00/52021 A1 discloses that sulfofucosylacylglycerol derivatives represented by the following formula are useful as DNA synthase inhibitors and carcinostatic agents

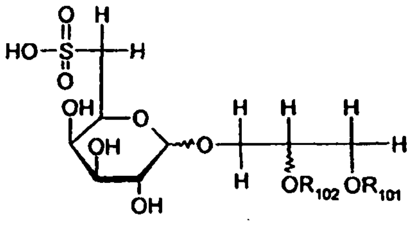

WO 96/34872A discloses that iodine derivatives of monosaccharides represented by the following formula are useful as a radiopharmaceutical product

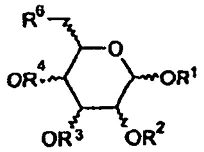

JP 57-67518A discloses a radiosensitizer containing as an active principle 3'-deoxyguanosine-5'-monophosphate, 3'-doxyadenosine-5'-rnonophosphate or their pharmaceutical acceptable salts.
JP 57-35516A discloses a radiosensitizer containing an active principle 3'-deoxyguanosine or 3'-deoxyuridine.
JP 2000-212087 discloses a radiosensitizeer represented by the following formula containing as an active principle a nitro-5-deazaflavin derivative.

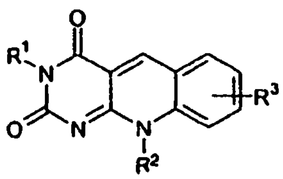

Disclosure of the Invention

The present invention has been made in view of the aforementioned problems, and a first object of the present invention is to provide a radiosensitizer which can be put into practice in combination with irradiation.
A second object of the present invention is to provide an anti-tumor radiation therapy method using the radiosensitizer.
In order to solve the aforementioned problems, the present inventors have intensively studied, and as a result, have found out that a sulfopyranosylacylglycerol derivative represented by the general formula (1) has the excellent radiosensitizing effect, which resulted in completion of the present invention. That is, the present invention provides the following radiosensitizer for use in combination with irradiation in an anti-tumor radiation treating method.

[1]. A radiosensitizer comprising, as an active ingredient, at least one kind of compound selected from the group

consisting of a compound represented by the following general formula (1):

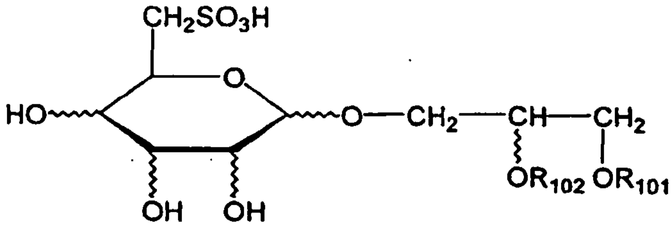

(1)

wherein $R_{101}$ represents an acyl residue of higher fatty acid, and $R_{102}$ represents a hydrogen atom or an acyl residue of higher fatty acid, and a pharmaceutically acceptable salt thereof.

[2] The radiosensitizer described in [1], wherein the active ingredient is at least one kind of compound selected from the group consisting of a compound represented by the following general formula (2):

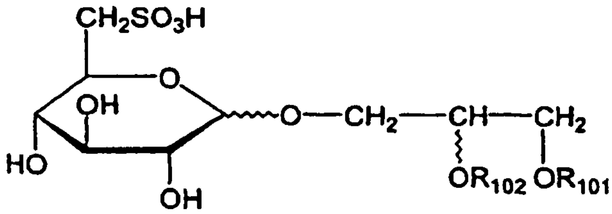

(2)

wherein $R_{101}$ represents an acyl residue of higher fatty acid, and $R_{102}$ represents a hydrogen atom or an acyl residue of higher fatty acid, and a pharmaceutically acceptable salt thereof.

[3] The radiosensitizer described in [2], wherein $R_{101}$ is R-CO- where R is an alkyl group having 13 to 25 carbon atoms, and $R_{102}$ is a hydrogen atom or R-CO-where R is an alkyl group having 13 to 25 carbon atoms in the general formula (2).

[4] The radiosensitizer described in [3], wherein $R_{101}$ is R-CO- where R is a straight alkyl group having an odd carbon number of 13 to 25 in the general formula (2).

[5] The radiosensitizer described in [3], wherein $R_{102}$ is a hydrogen atom in the general formula (2).

[6] The radiosensitizer described in [4], wherein $R_{102}$ is a hydrogen atom in the general formula (2).

[7] The radiosensitizer described in [3], wherein $R_{102}$ is R-CO- where R is an alkyl group having 13 to 25 carbon atoms in the general formula (2).

[8] The radiosensitizer described in [4], wherein $R_{102}$ is R-CO- where R is an alkyl group having 13 to 25 carbon atoms in the general formula (2).

Also, the present invention provides an anti-tumor radiation treating method comprising using the above radiosensitizer in combination with irradiation.

The radiosensitizer of the present invention can attain the synergistic anti-tumor treating effect exceeding prediction by using it in combination with irradiation.

Brief Description of Drawings

FIG. 1 shows a relationship between test days and tumor volume.
FIG. 2 shows a relationship between test days and tumor volume.
FIG. 3 shows a relationship between test days and tumor volume.
FIG. 4 shows a relationship between test days and tumor volume.
FIG. 5 shows a relationship between test days and tumor volume.
FIG. 6 shows a relationship between test days and tumor volume.
FIG. 7 shows a relationship between test days and tumor volume.
FIG. 8 shows results of colony assay.

FIG. 9 shows results of colony assay.
FIG. 10 shows results of colony assay.
FIG. 11 shows results of colony assay.
FIG. 12 shows results of colony assay which was performed by changing a radiation dose and a concentration of the radiosensitizer of the present invention.
FIG. 13 shows results of colony assay.
FIG. 14 shows results of colony assay.
FIG. 15 shows results of colony assay.
FIG. 16 shows results of colony assay.
FIG. 17 shows results of colony assay.
FIG. 18 shows results of colony assay.
FIG. 19 shows results of colony assay.
FIG. 20 shows results of assay using an angiogenesis kit.

Best Mode for Carrying Out the Invention

In the present specification, the radiosensitizer includes a radiosensitizer which can enhance the anti-tumor effect more than irradiation alone by using the sensitizer in combination with irradiation. The anti-tumor effect includes the case where a tumor is shrunk or is eradicated. In addition, the anti-tumor effect includes the case where growth of a tumor is delayed, and the case where the same anti-tumor effect can be attained at a smaller radiation dose or a fewer number of dose fractions, but it is not intended to restrict mechanism of action of the radiosensitizer of the present invention.
First, detailed description will be given of a sulfopyranosylacylglycerol derivative represented by the general formula (1):

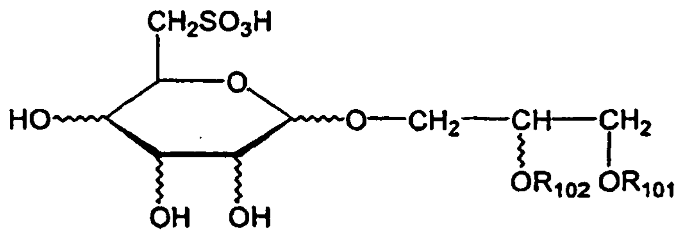

(1)

wherein $R_{101}$ represents an acyl residue of higher fatty acid, and $R_{102}$ represents a hydrogen atom or an acyl residue of higher fatty acid, which is an active ingredient of the radiosensitizer of the present invention.
In the sulfopyranosylacylglycerol derivative of the general formula (1), examples of pyranose which is a sugar skeleton constituting pyranoside include α-D-glucose, β-D-glucose, α-D-galactose, β-D-galactose, α-D-mannose, ß-D-mannose, and the like.
The sugar skeleton of the pyranoside can have any conformation of a boat-form and a chair-form. However, a chair-form is preferable from a viewpoint of stability.
An absolute configuration at a 2-positional carbon (asymmetric carbon) of a glycerol moiety may be any of S- and R-configuration.
In the general formula (1), $R_{101}$ represents an acyl residue of higher fatty acid. Examples of fatty acid giving the acyl residue of higher fatty acid represented by $R_{101}$ include straight or branched, saturated or unsaturated higher fatty acids.
The acyl residue of straight or branched higher fatty acid represented by $R_{101}$ includes a group represented by R-C(=O)- wherein R represents an alkyl or alkenyl group having 13 carbon atoms or more. A carbon number of an alkyl group and an alkenyl group represented by R of the acyl residue R-C(=O)- is preferably 13 or more and 25 or less, further preferably an odd number of 13 to 25, in view of the manufacturing cost, radiosensitizing activity and the like.
In the general formula (1), $R_{102}$ represents a hydrogen atom or an acyl residue of higher fatty acid. The acyl residue of higher fatty acid represented by $R_{102}$ has the same meaning as that of the acyl residue of higher fatty acid of the aforementioned $R_{101}$.
When both of $R_{101}$ and $R_{102}$ are an acyl residue of higher fatty acid in the general formula (1), these acyl residues may be the same or different, and are preferably the same from a viewpoint of easiness of manufacturing.
Examples of the sulfopyranosylacylglycerol derivative of the general formula (1) include a compound in which pyranose as a sugar skeleton constituting pyranoside is α-D-glucose, β-D-glucose, α-D-galactose, ß-D-galactose, α-D-mannose, or β-D-mannose; $R_{101}$ is a group represented by R-C(=O)-wherein R represents an alkyl group or an alkenyl

group having an odd carbon number of 13 to 25; $R_{102}$ is a hydrogen atom or an acyl residue of higher fatty acid having the same meaning as that of the $R_{101}$.

The sulfopyranosylacylglycerol derivative represented by the general formula (1) is the known compound, and can be prepared, for example, according to the method described in patent applications of the present applicant (Jpn. Pat. Appln. KOKAI Publication No.2000-143516, International Publication Nos. WO 00/52020, WO 00/52021, WO 00/51622 and the like).

Examples of a pharmaceutically acceptable salt of the sulfopyranosylacylglycerol derivative represented by the general formula (1) include, but not limited to, a salt of a monovalent cation such as sodium or potassium ion.

The radiosensitizer of the present invention contains, as an active ingredient, one or more kinds selected from the group consisting of the sulfopyranosylacylglycerol derivative represented by the general formula (1) of the present invention and a pharmaceutically acceptable salt thereof. As described above, examples of the sulfopyranosylacylglycerol derivative represented by the general formula (1) include a steric isomer of a pyranosyl moiety, an isomer at a C2 carbon (asymmetric carbon) of a glyceridyl moiety, and the like. The radiosensitizer of the present invention can contain these isomers alone, or a mixture of two or more kinds of isomers, as far as the activity of the radiosensitizer is not adversely influenced. Alternatively, the radiosensitizer of the present invention can contain plural kinds of compounds having different substituents $R_{101}$ and/or $R_{102}$ in the general formula (1). Further, the radiosensitizer of the present invention may be used in combination with another radiosensitizer, an anti-cancer agent, or another pharmacological active compound, as far as the activity of the radiosensitizer is not adversely influenced.

Hereinafter, a compound of the group consisting of the sulfopyranosylacylglycerol derivative represented by the general formula (1) of the present invention and a pharmaceutical acceptable salt thereof is also referred to as "the present radiosensitizing substance".

The present radiosensitizing substance can be administered, for example, orally or parenterally. The present radiosensitizing substance can be formulated into a pharmaceutical preparation by combining with suitable pharmaceutically acceptable excipients, diluents or the like, depending on these administration routes.

A dosage form suitable for oral administration includes forms of the state of a solid, a semisolid, a liquid, a gas or the like. Specifically, examples of the dosage form include, but not limited to, tablets, capsules, powders, granules, solutions, suspensions, syrups, elixirs or the like.

For formulating the present radiosensitizing substance into tablets, capsules, powders, granules, solutions, suspensions or the like, formulation can be performed by mixing the present radiosensitizing substance with a binder, a tablet disintegrating agent, a lubricant or the like, and further, if necessary, mixing with a diluent, a buffer, a wetting agent, a preservative, a flavor or the like, using the known per se method. By way of one example, the binder includes crystalline cellulose, cellulose derivatives, corn starch, gelatin and the like; the tablet disintegrating agent includes corn starch, potato starch, sodium carboxymethylcellulose and the like; and the lubricant includes talc, magnesium stearate and the like. Further, the conventionally used additives such as lactose, mannitol and the like, can be used.

Alternatively, the present radiosensitizing substance may be administered in a form of aerosol or inhalant by filling the substance of liquid- or fine powder-form together with a gaseous or liquid spraying agent, and if necessary, a known auxiliary agent such as a wetting agent, into a non-pressurized container such as an aerosol container or a nebulizer. As the spraying agent, a pressurized gas such as dichlorofluoromethane, propane, nitrogen or the like, can be used.

When the present radiosensitizing substance is administered parenterally, the substance can be administered by, for example, injection, transdermal administration, rectal administration, intraocular administration, or so on.

As the administration by injection, the substance can be administered subcutaneously, intradermally, intravenously, intramuscularly or so on. These injection preparations can be obtained by dissolving, suspending or emulsifying the present radiosensitizing substance in an aqueous or non-aqueous solvent such as vegetable oil, synthetic fatty acid glyceride, higher fatty acid ester, or propylene glycol, and further, if desired, formulating this together with a conventional additive such as a solubilizing agent, an osmoregulating agent, an emulsifier, a stabilizer or a preservative into preparations, by the known per se method.

For formulating the present radiosensitizing substance into a form of solutions, suspensions, syrups, elixirs or the like, a pharmaceutically acceptable solvent such as sterilized water for injection and normal physiological saline can be used.

For transdermal administration, the substance can be administered as ointments, emulsions, pastes, cataplasms, liniments, lotions, suspensions or the like, depending on the state of a skin to be administered.

Ointments can be formulated into preparations by kneading the present radiosensitizing substance together with a hydrophobic base such as Vaseline or paraffin, or with a hydrophilic base such as hydrophilic Vaseline or macrogol by the known per se method. Emulsions and other transdermal agents may be formulated into preparations by the conventionally used method.

For rectal administration, for example, the substance can be administered as suppositories. Suppositories can be formulated into preparations by mixing the present radiosensitizing substance together with an excipient such as

cacao butter, carbon wax, or polyethylene glycol which is melted at a body temperature but is solid at room temperature, and forming the mixture, by the known per se method.

For intraocular administration, the substance can be administered as ophthalmic preparations such as eye drops or ophthalmic ointments. Eye drops can be formulated into preparations by dissolving or suspending the present radiosensitizing substance in an aqueous solvent such as sterilized purified water, and if necessary, adding a preservative, a buffer, a surfactant or the like, by the known per se method.

An administration condition (e.g. dose, the number of dosing times and dosing interval) for the present radiosensitizing substance can be appropriately set and regulated depending on an administration form, an administration route, a condition of target tumor (e.g. a type, a site and a progress stage of tumor), a condition of radiation therapy (e.g. a type, a dose and the number of irradiation times of radiation ray), how to use in combination with radiation therapy (e.g. period of radiation therapy, and order of administration of the present radiosensitizer) and the like. By way of one example, in the case of oral administration, a dose of the radiosensitizing substance can be set to be 0.001 to 100 mg/kg body weight/day; in the case of administration by injection, a dose of the radiosensitizing substance can be set to be 0.001 to 50 mg/kg body weight/day; in the case of transdermal administration, a dose of the radiosensitizing substance can be set to be 0.001 to 100 mg/kg body weight/day; in the case of rectal administration, a dose of the radiosensitizing substance can be set to be 0.001 to 50 mg/kg body weight/day; and in the case of intraocular administration, the radiosensitizing substance can be administered in a form of eye drops by dividing about 0.001 to 3% of the eye drops solution into a few times per day, but the administration condition is not limited thereto.

On the other hand, with respect to radiation therapy, a type, a dose and the number of dose fractions can be the same condition as those of the conventionally performed radiation therapy. Examples of the conventional radiations used for a human include medical radiations, for example, X-rays, γ-rays, electron beams and β-rays as well as particle beams such as π-mesons, neutrons and other heavy particles and the like, at an irradiation dose of about 0.1 to 100 Gy per fraction, at a total irradiation dose of about 10 to 500 Gy over a period of 1 week to 6 months. Representative examples of irradiation to a human include irradiation of X-ray at 2 Gy per fraction, five times a week, at a total of 60 Gy over a period of about 6 weeks, but the irradiation condition is not limited thereto. For example, an irradiation dose or the number of irradiation times can be reduced. Further, the irradiation can be performed by conformal radiotherapy, stereotactic radiotherapy targeting a cancer lesion as a pin point, or intensity modulated radiotherapy. In addition, irradiation may be performed by irradiation with a sealed small radiation source, γ-ray teleirradiation, or irradiation with a particle beam. Internal irradiation makes it possible to increase an irradiation dose per time and shorten an irradiation period.

Irradiation and administration of the present radiosensitizer may be performed over the same period, or any of them may precede the other. In this case, it is expected that the present radiosensitizer serves as an anti-tumor agent to be used in combination with irradiation, or as an angiogenesis suppressing agent to be used in combination with irradiation.

The aforementioned irradiation condition for radiation and the aforementioned administration condition for the present radiosensitizer can be appropriately selected by a medical practitioner or other professionals, depending on a type of a radiation source, an irradiation method, an irradiation site and an irradiation period; a type of the sensitizer, an administration route and an administration period; a disease to be treated and severity of the disease; an age, a weight, health state and disease history of a subject to be irradiated, as is well-known in the field of radiation therapy.

## Examples

Examples of the present invention will be explained below, but the present invention is not limited thereto.

### <Example 1 (animal experiment)>

(Experimental Example 1-1)

Nude mice were assigned to four groups (control group; single use group of the present radiosensitizer administration; single use group of irradiation; and combined use group of irradiation and the present radiosensitizer administration; 4 mice per group). 1.0 x $10^6$ human tongue squamous carcinoma cells (SAS cells) were suspended in PBS(-), and the suspension was transplanted subcutaneously into a right thigh of each mouse. After 10 to 14 days, when tumor volume became a desired value in a range of about 50 $mm^3$ to about 100 $mm^3$, each mouse was subjected to treatment according to each group. As a radiosensitizer, 3-O-(6-deoxy-6-sulfo-a-D-qlucopyranosyl) -I-O-stearoyl-glycerol sodium salt (hereinafter, also referred to as "α-SQMG C18:0") was used. Regarding the single use group of irradiation, X-ray was irradiated two times (at the time of treatment initiation (day 0) and on day 6 after the treatment initiation) at a dose of 8 Gy. Regarding the single use group of α-SQMG C18:0 administration, the sensitizer was intraperitoneally administered two times (day 0 and day 6) at a concentration of 1 mg/kg. Regarding the combined use group, each mouse of

the group was treated with both of irradiation and α-SQMG C18:0 administration. Regarding the control group, neither irradiation nor α-SQMG C18:0 administration was performed. Thereafter, a short diameter and a long diameter of a tumor were measured with a micrometer caliper over a period of 16 to 23 days. Tumor volume was calculated by the following equation, and the tumor growth delaying effect was compared.

Equation for calculating tumor volume:

$$\text{tumor volume } (mm^3) = (\text{short diameter})^2 \times (\text{long diameter}) \times 0.5$$

The obtained results are shown in FIG. 1. In FIG. 1, the horizontal axis indicates the number of days after treatment initiation, and the vertical axis indicates tumor volume. In addition, an open arrow indicates a day on which X-ray was irradiated, and a closed arrow indicates a day when α-SQMG C18:0 was administered. In the following FIGS. 2 to 7, open and closed arrows have the same meanings.

From the results shown in FIG. 1, the anti-tumor effect far exceeding the anti-tumor effect attained by irradiation, was obtained by using α-SQMG C18:0 administration in combination with irradiation.

(Experimental Example 1-2)

The same experiment as that of Experimental Example 1-1 was performed except that irradiation was performed at the time of treatment initiation (day 0) and on day 4 after the treatment initiation, administration of α-SQMG C18:0 was performed only on day 0, and measurement of tumor size was performed over a period of 26 days.

The obtained results are shown in FIG. 2. From the results shown in FIG. 2, the anti-tumor effect far exceeding the anti-tumor effect attained by irradiation, was obtained by using α-SQMG C18:0 administration in combination with irradiation.

In order to express numerically this synergistic effect, ER (enhancement ratio) was calculated by the following equation. ER is a value defined by the literature (Int., J. Radiation Oncology Bio. Phys. Vol. 55, No. 3, pp. 713-723 (2003)), and ER will be explained below based on the definition.

$$ER = NGD \div TGD \text{ in the case of X-ray irradiation alone}$$

In the equation, "TGD (tumor growth delay)" indicates delay of days necessary for tumor volume to reach a predetermined size in each test group as compared with a control group. That is, this is expressed by the following equation. In the Examples of the present invention, the predetermined size of tumor volume is 500 $mm^3$, unless otherwise indicated.

$$TGD = (\text{number of days necessary for tumor volume to reach } 500\ mm^3 \text{ in test group}) - (\text{number of days necessary for tumor volume to reach } 500\ mm^3 \text{ in control group})$$

"NGD (normalized growth delay)" is expressed by the following equation.

$$NGD = (\text{TGD in the case of combined use}) - (\text{TGD in the case of drug administration alone})$$

According to the aforementioned literature, it can be said that the anti-tumor effect is synergistic, when an enhancement ratio (ER) obtained by the above equation exceeds 1.0.

As a result of the animal experiment of Experimental Example 1-2, an enhancement ratio (ER) is 2.0, and it is shown that synergistic effect is very high.

(Experimental Example 1-3)

The same experiment as that of Experimental Example 1-1 was performed except that irradiation was performed at the time of treatment initiation (day 0) and on day 3 after the treatment initiation, α-SQMG C18:0 was administered once a day from day 0 to day 4, and measurement of tumor size was performed over a period of 35 days.

The obtained results are shown in FIG. 3. From the results shown in FIG. 3, the anti-tumor effect far exceeding the anti-tumor effect attained by irradiation, was obtained by using administration of α-SQMG C18:0 in combination with irradiation.

In the animal experiment of Experimental Example 1-3, an enhancement ratio (ER), which is obtained by the equation described in Experimental Example 1-2, is 3.0, and it is shown that synergistic effect is extremely high.

(Experimental Example 1-4)

In the present Experimental Example, an experiment was performed in the same manner as in the above Experimental Example except that irradiation was performed at a dose of 2 Gy per fraction everyday from the time of treatment initiation (day 0) to day 4, and administration of α-SQMG C18:0 was performed everyday from day 0 to day 4. Details of the experiment will be described below.

Four nude mice (eight weeks old) were randomly assigned to each of four groups. 1.0 x $10^6$ human tongue squamous carcinoma cells (SAS cells) were suspended in PBS (-), and the suspension was transplanted subcutaneously into a right thigh of each mouse. After 10 days, when tumor volume became about 50 $mm^3$, each mouse was subjected to treatment according to each group. As a radiosensitizer, α-SQMG C18:0 was used as in the aforementioned Experimental Examples. Regarding a single use group of irradiation, X-ray was irradiated at a dose of 2 Gy once a day at a total of 5 times from the time of treatment initiation (day 0) to day 4. Regarding a single use group of α-SQMG C18:0 administration, the sensitizer was intraperitoneally administered at a concentration of 1 mg/kg once a day at a total of 5 times from the time of treatment initiation (day 0) to day 4. Regarding the combined use group, each mouse of the group was treated with both irradiation and α-SQMG C18:0 administration. Regarding a control group, neither irradiation nor α-SQMG C18:0 administration was performed. Thereafter, a short diameter and a long diameter of a tumor were measured with a micrometer caliper over a period of 30 days. Tumor volume was calculated according to the following equation, and the tumor growth delaying effect was compared.

Equation for calculating tumor volume:

$$\text{tumor volume } (mm^3) = (\text{short diameter})^2 \times (\text{long diameter}) \times 0.5$$

The obtained results are shown in FIG. 4. From the results shown in FIG. 4, even when the same dose (2 Gy per time) as that normally used in the clinical field was irradiated five times, and a total irradiation dose was 10 Gy, it was found that there was the anti-tumor effect exceeding the anti-tumor effect attained by irradiation alone.

In order to express numerically this synergistic effect, an enhancement ratio (ER) was calculated according to the method described in Experimental Example 1-2. As a result, ER was 1.8, and it was found that synergistic effect was high.

The present Experimental Example is compared with the Experimental Example 1-3 (in which an irradiation dose of 8 Gy per time was irradiated at two fractions and a total irradiation dose was 16 Gy). Although, in the present Experimental Example, an irradiation dose per fraction was smaller than that of Experimental Example 1-3, and a total irradiation dose was smaller, the synergistic anti-tumor effect was confirmed as is the case with the Experimental Example 1-3.

(Experimental Example 1-5)

Five nude mice (eight weeks old) were randomly assigned to each of four groups. Human esophageal cancer cells (TE-8 cells) were transplanted to each mouse in the same manner as the above Experimental Example 1-1. When

tumor volume reached 50 $mm^3$, α-SQMG C18:0 administration and irradiation treatment were performed. In a single use group of irradiation, each mouse was irradiated with X-ray at a dose of 8 Gy two times on day 0 and day 4. In a single use group of α-SQMG C18:0 administration, the sensitizer was intraperitoneally administered at a concentration of 1 mg/kg once a day at a total of 5 times from day 0 to day 4. In a combined use group, each mouse of the group was treated with both of irradiation and α-SQMG C18:0 administration. In the control group, neither irradiation nor α-SQMG C18:0 administration was performed. Thereafter, tumor volume was measured over a period of 51 days in the same manner as the above Experimental Example 1-1.

The obtained results are shown in FIG. 5. From the results shown in FIG. 5, also regarding human esophageal cancer, the anti-tumor effect far exceeding the anti-tumor effect attained by irradiation, was obtained by using α-SQMG C18:0 in combination with irradiation. In addition, an enhancement ratio (ER) was obtained according to the method described in Experimental Example 1-2. In the present Experimental Example, the anti-tumor effect was extremely high, and tumor volume in the combined use group did not reach 500 $mm^3$ by completion of the experiment. For this reason, an enhancement ratio was calculated from the number of days until tumor volume reaches 400 $mm^3$. As a result, ER was 3.25, and it was shown that synergistic effect was extremely high.

(Experimental Example 1-6)

A human cancer-transplanted mouse was prepared in the same manner as in Experimental Example 1-5 except that human uterine cervical cancer cells (HeLa cells) were transplanted into a nude mouse. When tumor volume reached 100 $mm^3$, α-SQMG C18:0 administration and irradiation treatment were performed. In a single use group of irradiation, each mouse was irradiated with X-ray at a dose of 8 Gy two times on day 0 and day 4. In a single use group of α-SQMG C18:0 administration, α-SQMG C18:0 was intraperitoneally administered at a concentration of 1 mg/kg at a total of 10 times consisting of two courses of 5 times, that is, once a day from day 0 to day 4 and once a day from day 12 to day 16. In the combined use group, each mouse of the group was treated with both of irradiation and α-SQMG C18:0 administration. In the control group, neither irradiation nor α-SQMG C18:0 administration was performed. Thereafter, tumor volume was measured over a period of 35 days in the same manner as the above Experimental Example 1-1.

The obtained results are shown in FIG. 6. From the results shown in FIG. 6, also regarding a human uterine cervical cancer, the anti-tumor effect far exceeding the anti-tumor effect attained by irradiation, was obtained by using α-SQMG C18:0 in combination with irradiation. In addition, an enhancement ratio (ER) was obtained according to the method described in Experimental Example 1-2. As a result, ER was 2.71, and it was shown that synergistic effect was extremely high.

(Experimental Example 1-7)

In the present Experimental Example, an experiment was performed in the same manner as in the aforementioned Experimental Examples except that human lung cancer cells (A549 cells) were transplanted into a nude mouse. Details of the experiment will be described below.

Four nude mice (eight weeks old) were randomly assigned to each of four groups. $2.0 \times 10^6$ human lung cancer cells (A549 cells) were suspended in PBS (-), and the suspension was transplanted subcutaneously into a right thigh of each mouse. After 45 days, when tumor volume became about 50 $mm^3$, each mouse was subjected to treatment according to each group. As a radiosensitizer, α-SQMG C18:0 was used as in the aforementioned Experimental Examples. In a single use group of irradiation, X-ray was irradiated two times (at the time of treatment initiation (day 0) and on day 3 after the treatment initiation) at a dose of 4 Gy. In a single use group of α-SQMG C18:0 administration, α-SQMG C18:0 was intraperitoneally administered at a concentration of 1 mg/kg once a day at a total of 5 times from the time of treatment initiation (day 0) to day 4. In a combined use group, each mouse of the group was treated with both of irradiation and α-SQMG C18:0 administration. In a control group, neither irradiation nor α-SQMG C18:0 administration was performed. Thereafter, a short diameter and a long diameter of a tumor were measured with a micrometer caliper over a period of 20 days. Tumor volume was calculated according to the following equation, and the tumor growth delaying effect was compared.

Equation for calculating tumor volume:

$$\text{tumor volume } (mm^3) = (\text{short diameter})^2 \times (\text{long diameter}) \times 0.5$$

The obtained results are shown in FIG. 7. From the results shown in FIG. 7, also regarding a lung cancer which is an adenocarcinoma, it was found that there was the anti-tumor effect exceeding the anti-tumor effect attained by

irradiation alone, by using irradiation together with α-SQMG C18:0 administration.

In the present Experimental Example, growth of a tumor is suppressed in the combined use group, and increase in tumor volume is scarcely seen. For this reason, an enhancement ratio (ER) value for expressing this synergistic effect cannot be calculated. In other words, results of the present Experimental Example demonstrate that the synergistic effect of α-SQMG C18:0 administration and irradiation is high to the extent that calculation of an ER value is impossible.

As described above, the radiosensitizer represented by the general formula (1) of the present invention exhibits such a high synergistic anti-tumor effect. Thus, it is shown that the radiosensitizer represented by the general formula (1) of the present invention can attain the anti-tumor effect equivalent to the effect expected by the present radiosensitizing substance alone or the irradiation alone, at a smaller dose or a smaller total radiation amount. As a result, it is expected that the incidence of the side effect which can be normally caused by chemotherapeutic drug or irradiation is reduced by using the present radiosensitizer.

In addition, throughout the aforementioned experiments, there was no change in a weight of each nude mouse. Further, when the present radiosensitizer was applied on a skin of a mouse, there was neither inflammation nor other problem. Moreover, inflammation of a skin caused by irradiation was not recognized. Also from these facts, it is presumed that the present radiosensitizer has few side effects.

In the aforementioned Examples, as the present radiosensitizer, α-SQMG C18:0 (3-O-(6-deoxy-6-sulfo-a-D-glucopyranosyl)-1-O-stearoyl-glycerol sodium salt) was used. However, it can be expected that equal or more anti-tumor effect is obtained, also when other compound represented by the general formula (1) is used (for example, saturated monoacylglyceride having a myristoyl group or a palmitoyl group or other saturated acyl group instead of a stearoyl group of α-SQMG C18:0; unsaturated monoacylglyceride having $CH_3$-$(CH_2)_3$-$(CH=CH-CH_2)_1$-$(CH_2)_6$-CO-, $CH_3$-$(CH_2)_5$-$(CH=CH-CH_2)_1$-$(CH_2)_6$-CO-, $CH_3$-$(CH_2)_7$-$(CH=CH-CH_2)_1$-$(CH_2)_6$-CO- or other unsaturated acyl group; or saturated/unsaturated diacylglyceride having any one or two kinds selected from these six kinds of acyl groups and other acyl groups). In the following Experimental Examples 2-6 to 2-12, the effect as the radiosensitizer has been confirmed in the following compounds of the general formula (1): a compound of the general formula (1) wherein a myristoyl group, a palmitoyl group or an oleoyl group is possessed in place of a stearoyl group of α-SQMG C18:0; a compound of the general formula (1) wherein a bond between pyranose and glycerol is a β bond in place of an α bond; a compound of the general formula (1) which is a diacyl derivative in place of a monoacyl derivative; and a compound of the general formula (1) which has a sugar skeleton other than glucose. Therefore, it can be expected that equal or more anti-tumor effect is obtained, also when other compounds represented by the general formula (1) are used.

In the above Example, the effect of the sensitizer of the present invention on tongue squamous cell carcinoma, esophageal cancer, uterine cervical cancer and lung cancer was demonstrated. However, it can be expected that equal or more anti-tumor effect of the sensitizer of the present invention is exerted in other cancers (e.g. adenocarcinoma such as prostate cancer, colon cancer, stomach cancer, mammary gland cancer, pancreas cancer, liver cancer, neuroglia cancer, and cervical adenocarcinoma, solid cancer such as transitional cell cancer, sarcoma and melanoma, and humoral cancer such as lymphoma).

Further, it can be expected that the equal or more anti-tumor effect is exerted in the cases other than the aforementioned condition, by appropriately changing dose and the number of dosing of the present radiosensitizer; a kind, an irradiation dose, and the number of dose fractions; and an administration order of irradiation and the present radiosensitizer. Such the condition setting can be performed according to a procedure which is normally performed by doctors or other medical practitioners.

<Example 2 (colony assay)>

(Experimental Example 2-1)

Bovine aortic endothelial cells (BAEC) were appropriately seeded on a petri dish, and adhesion of cells was confirmed after 2 to 3 hours. In a group of α-SQMG C18:0 administration alone and a combined use group, the cells were treated with 10 μM of α-SQMG C18:0. After 24 hours, a medium was exchanged in all test groups, and 4 Gy of $^{60}$Co γ-ray was irradiated in a group of irradiation alone and a combined use group. After the cells were cultured in a $CO_2$ incubator at 37°C for 10 to 12 days, formed colonies were fixed with formalin, and stained with a crystal violet staining solution. On each plate, the number of colonies consisting of about 50 or more cells was counted, and cell surviving fraction (SF) was obtained according to the following equation.

SF (cell surviving fraction) = number of colonies/number of seeded cells × (PE/100)

Herein, PE (plating efficiency) represents a colony formation rate (%) on a plate of a control.

An experiment was performed three times, and an average of them was calculated.

As a result of the experiment, SF values in the group of α-SQMG C18:0 administration alone and the group of 4 Gy irradiation alone were 0.616 and 0.158, respectively. FIG. 8 shows a theoretical additive point of combined use (SF value in group of α-SQMG C18:0 administration alone x SF value in group of 4 Gy irradiation alone) calculated from the above SF values, and an actually measured SF value of the combined use group.

A theoretical additive point of combined use was 0.0974, while an actually measured SF value of a combined use group was 0.0640, which was greatly below the theoretical additive point.

(Experimental Example 2-2)

The same experiment as that of Experimental Example 2-1 was performed except that a radiation dose 6 Gy was irradiated in place of 4 Gy used in Experimental Example 2-1.

As a result of the experiment, SF values in the group of α-SQMG C18:0 administration alone and the group of 6 Gy irradiation alone were 0.616 and 0.0820, respectively. FIG. 9 shows a theoretical additive point of combined use (SF value in group of α-SQMG C18:0 administration alone x SF value in group of 6 Gy irradiation alone) calculated from the above SF values, and an actually measured SF value of the combined use group.

A theoretical additive point of combined use was 0.0505, while an actually measured SF value of a combined use group was 0.0180, which was greatly below the theoretical additive point.

In parallel with the Experimental Examples 2-1 and 2-2, the same colony assay on BAEC was performed also on SAS cells, but α-SQMG C18:0 did not exert a cell proliferation suppressing effect on the SAS cells.

From the results of the Experimental Examples 2-1 and 2-2, it was demonstrated that the combined use of α-SQMG C18:0 and radiation exerted a considerably great synergistic effect on suppression of proliferation of vascular endothelial cells.

(Experimental Example 2-3)

The same experiment as that of Experimental Example 2-1 was performed except that a radiation dose 2 Gy was irradiated in place of 4 Gy used in Experimental Example 2-1. Results are shown in FIG. 10.

As a result of the experiment, SF values in a group of 10 μM α-SQMG C18:0 administration alone and a group of 2 Gy irradiation alone were 0.9433 and 0.5957, respectively. A theoretical additive point of combined use calculated from the above SF values was 0.5620, while an actually measured SF value in the case of combined use of α-SQMG C18:0 and irradiation was 0.0644, which was greatly below the theoretical additive point.

(Experimental Example 2-4)

The same experiment as that of Experimental Example 2-3 was performed except that cells were treated with 5 μM of α-SQMG C18:0 in place of 10 μM of α-SQMG C18:0 used in Experimental Example 2-3. Results are shown in FIG. 11.

As a result of the experiment, SF values in a group of 5 μM α-SQMG C18:0 administration alone and a group of 2 Gy irradiation alone were 1.0094 and 0.5957, respectively. A theoretical additive point of combined use calculated from the above SF values was 0.6012, while an actually measured SF value in the case of combined use of 5 μM α-SQMG C18:0 and irradiation was 0.1370, which was greatly below the theoretical additive point.

(Experimental Example 2-5)

In the present Experimental Example, various colony assays were performed according to the same manners as those of the Experimental Examples 2-1 to 2-4. That is, colony assays were performed by variously changing an administration concentration of α-SQMG C18:0 in a range of 0 to 25 μM and an irradiation dose of $^{60}$Co γ-ray in a range of 0 to 8 Gy, respectively.

A summary of results is shown in FIG. 12. In FIG. 12, each data indicated by "0 Gy", "2 Gy", "4 Gy", "6 Gy" and "8 Gy" shows the result of each experiment in which $^{60}$Co γ-ray was irradiated at an indicated irradiation dose, and an administration concentration of α-SQMG C18:0 was changed in a range of 0 to 25 μM. From these results, it can be seen that the cell proliferation suppressing effect is not obtained by treatment with α-SQMG C18:0 alone, but the effect of irradiation on suppression of cell proliferation is increased when administration of α-SQMG C18:0 is used in combination with irradiation.

(Experimental Example 2-6)

The same experiment as that of Experimental Example 2-2 was performed except that 3-O-(6-deoxy-6-sulfo-α-D-glucopyranosyl)-1-O-myristoyl-glycerol sodium salt (hereinafter, "α-SQMG C14:0") was used in place of α-SQMG C18:0 used in Experimental Example 2-2. Results are shown in FIG. 13.

As a result of the experiment, SF values in a group of α-SQMG C14:0 administration alone and a group of 6 Gy irradiation alone were 1.0605 and 0.0674, respectively. A theoretical additive point of combined use calculated from the above SF values was 0.0715, while an actually measured SF value in the case of combined use of α-SQMG C14:0 and irradiation was 0.0109, which was greatly below the theoretical additive point.

(Experimental Example 2-7)

The same experiment as that of Experimental Example 2-2 was performed except that 3-0-(6-deoxy-6-sulfo-α-D-glucopyranosyl)-1-O-palmitoyl-glycerol sodium salt (hereinafter, "α-SQMG C16:0") was used in place of α-SQMG C18:0 used in Experimental Example 2-2. Results are shown in FIG. 14.

As a result of the experiment, SF values in a group of α-SQMG C16:0 administration alone and a group of 6 Gy irradiation alone were 0.9553 and 0.0674, respectively. A theoretical additive point of combined use calculated from the above SF values was 0.0644, while an actually measured SF value in the case of combined use of α-SQMG C16:0 and irradiation was 0.0233, which was greatly below the theoretical additive point.

From the results of Experimental Example 2-6, it was demonstrated that the combined use of α-SQMG C14:0 and radiation exerted a considerably great synergistic effect on suppression of proliferation of vascular endothelial cells. From the results of Experimental Example 2-7, it was demonstrated that the combined use of α-SQMG C16:0 and radiation exerted a considerably great synergistic effect on suppression of proliferation of vascular endothelial cells.

(Experimental Example 2-8)

The same experiment as that of Experimental Example 2-1 was performed except that 3-O-(6-deoxy-6-sulfo-α-D-glucopyranosyl)-1-0-oleoyl-glycerol sodium salt (hereinafter, "α-SQMG C18:1") was used in place of α-SQMG C18:0 used in Experimental Example 2-1. Results are shown in FIG. 15.

As a result of the experiment, SF values in a group of α-SQMG C18:1 administration alone and a group of 4 Gy irradiation alone were 1.0392 and 0.1915, respectively. A theoretical additive point of combined use calculated from the above SF values was 0.1990, while an actually measured SF value in the case of combined used of α-SQMG C18:1 and irradiation was 0.0843, which was greatly below the theoretical additive point.

(Experimental Example 2-9)

The same experiment as that of Experimental Example 2-1 was performed except that 3-O-(6-deoxy-6-sulfo-β-D-glucopyranosyl)-1-0-stearoyl-glycerol sodium salt (hereinafter, "β-SQMG C18:0") was used in place of α-SQMG C18:0 used in Experimental Example 2-1. Results are shown in FIG. 16.

As a result of the experiment, SF values in a group of β-SQMG C18:0 administration alone and a group of 4 Gy irradiation alone were 0.8562 and 0.1915, respectively. A theoretical additive point of combined use calculated from the above SF values was 0.1640, while an actually measured SF value in the case of combined use of β-SQMG C18:0 and irradiation was 0.0922, which was greatly below the theoretical additive point.

(Experimental Example 2-10)

The same experiment as that of Experimental Example 2-1 was performed except that 3-O-(6-deoxy-6-sulfo-β-D-qlucopyranosyl)-1,2-di-O-stearoyl-glycerol sodium salt (hereinafter, "β-SQDG C18:0") was used in place of α-SQMG C18:0 used in Experimental Example 2-1. Results are shown in FIG. 17.

As a result of the experiment, SF values in a group of β-SQDG C18:0 administration alone and a group of 4 Gy irradiation alone were 0.7190 and 0.1915, respectively. A theoretical additive point of combined use calculated from the above SF values was 0.1377, while an actually measured SF value in the case of combined use of β-SQDG C18:0 and irradiation was 0.0908, which was greatly below the theoretical additive point.

(Experimental Example 2-11)

The same experiment as that of Experimental Example 2-1 was performed except that 3-O-(6-deoxy-6-sulfo-α-D-glucopyranosyl)-1,2-di-O-stearoyl-glycerol sodium salt (hereinafter, "α-SQDG C18:0") was used in place of α-SQMG

C18:0 used in Experimental Example 2-1. Results are shown in FIG. 18.

As a result of the experiment, SF values in the group of α-SQDG C18:0 administration alone and the group of 4 Gy irradiation alone were 0.9216 and 0.1915, respectively. A theoretical additive point of combined use calculated from the above SF values was 0.1765, while an actually measured SF value in the case of combined use of α-SQDG C18:0 and irradiation was 0.0882, which was greatly below the theoretical additive point.

(Experimental Example 2-12)

The same experiment as that of Experimental Example 2-1 was performed except that 3-O-(6-deoxy-6-sulfo-α-D-mannopyranosyl)-1-O-stearoyl-glycerol sodium salt (hereinafter, "α-SRMG C18:0") was used in place of α-SQMG C18:0 used in Experimental Example 2-1. Results are shown in FIG. 19.

As a result of the experiment, SF values in a group of α-SRMG C18:0 administration alone and a group of 4 Gy irradiation alone were 0.8758 and 0.1915, respectively. A theoretical additive point of combined use calculated from the above SF values was 0.1677, while an actually measured SF value in the case of combined use of α-SRMG C18:0 and irradiation was 0.0784, which was greatly below the theoretical additive point.

The vascular endothelial cell used in the Examples is a cell which forms a new blood vessel newly reaching a tumor mass generated in a living body. A new blood vessel causes the proliferation of a tumor by supplying a nutrient and oxygen to a tumor cell via the blood vessel. Thus, suppression of proliferation of the vascular endothelial cell is tightly associated with the anti-tumor effect. Therefore, the results of the aforementioned colony assay significantly support the anti-tumor effect of the present radiosensitizer.

It is said that an angiogenesis inhibitor having the action of inhibiting the formation of a blood vessel has an advantage that acquisition of resistance is little, the uniform effect is obtained irrespective of a kind of a tumor, and the side effect is slight.

<Example 3 (assay by angiogenesis kit)>

The anti-angiogenic effect obtained by combined use of 20 μM of α-SQMG C18:0 administration and 4 Gy of $^{60}$Co γ-ray irradiation was investigated using an angiogenesis kit ("angiogenesis kit KZ-1000" of Kurabo Industries Ltd.).

The results obtained by the angiogenesis kit are quantified with imaging software, and they are shown in FIG. 20.

A drug named Suramin is the known substance as a drug suppressing angiogenesis, and it is known that its action is to inhibit a growth factor receptor of a vascular endothelial cell. In the present Example, Suramin was used as a positive control at 50 μM. [0112]

In the case of use of α-SQMG C18:0 alone (tube formation rate 0.7229), the anti-angiogenic effect was considerably lower as compared with the case of use of Suramin alone (tube formation rate 0.3386). However, when α-SQMG C18:0 was used in combination with radiation, the effect was higher than the case of combined use of Suramin and radiation. It was found that angiogenesis is synergistically suppressed by combined use of α-SQMG C18:0 and radiation.

As described above, the "theoretical additive point of combined use" is calculated by multiplication, and the theoretical value is compared with an actually measured value.

| | 4 Gy+Suramin | 4 Gy+α-SQMG |
|---|---|---|
| Theoretical value | 0.219 | 0.467 |
| Actually measured value | 0.232 | 0.0762 |

An actually measured value of a tube formation rate in the case of combined use of radiation and Suramin was slightly higher than the theoretical value. However, an actually measured value of a tube formation rate in the case of radiation and α-SQMG was greatly below the theoretical additive point of combined use. This result demonstrates that the anti-angiogenic effect obtained by the combined use of radiation and α-SQMG is higher than the theoretical value.

Proliferation of a tumor generated in a living body is accompanied with new formation of a blood vessel for supplying a nutrient and oxygen to a tumor cell. Therefore, the anti-angiogenic effect is tightly associated with the anti-tumor effect. Hence, the result of Example 3 significantly supports the anti-tumor effect of the present radiosensitizer.

## Claims

1. Use of at least one kind of compound selected from the group consisting of a compound represented by the following general formula (1):

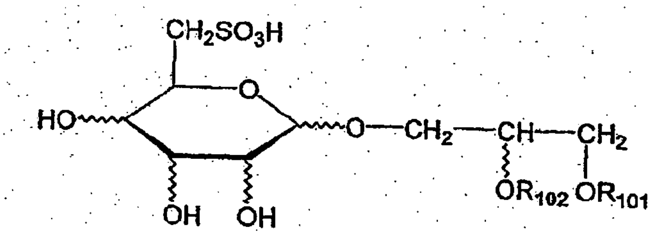

(1)

wherein $R_{101}$ represents an acyl residue of higher fatty acid, and $R_{102}$ represents a hydrogen atom or an acyl residue of higher fatty acid, and
a pharmaceutically acceptable salt thereof
for the preparation of a radiosensitizer for use in combination with irradiation in an anti-tumor radiation treating method.

2. The use according to claim 1, wherein the compound is selected from the group consisting of a compound represented by the following general formula (2):

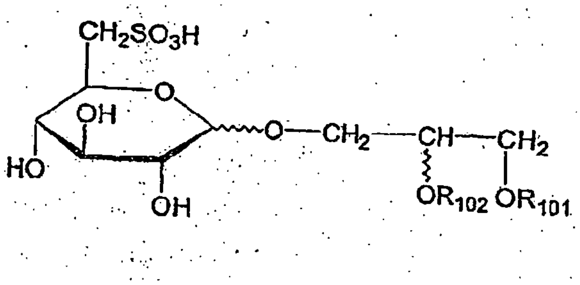

(2)

wherein $R_{10}$, represents an acyl residue of higher fatty acid, and $R_{102}$ represents a hydrogen atom or an acyl residue of higher fatty acid, and a pharmaceutically acceptable salt thereof

3. The use according to claim 2, wherein $R_{101}$ is R-CO- wherein R is an alkyl group having 13 to 25 carbon atoms, and $R_{102}$ is a hydrogen atom or R-CO- where R is an alkyl group having 13 to 25 carbon atoms in the general formula (2)

4. The use according to claim 3, wherein $R_{101}$, is R-CO- where R is a straight alkyl group having an odd carbon number of 13 to 25 in the general formula (2).

5. The use according to claim 3 or 4, wherein $R_{102}$ is a hydrogen atom in the general formula (2).

6. The use according to claim 3 or 4, wherein $R_{102}$ is R-CO= where R is an alkyl group having 13 to 25 carbon atoms in the general formula (2).

7. At least one kind of compound selected from the group consisting of a compound represented by the following general formula (1):

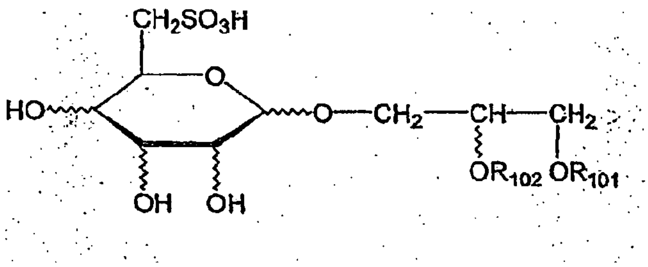

(1)

wherein $R_{101}$ represents an acyl residue of higher fatty acid, and $R_{102}$ represents a hydrogen atom or an acyl residue of higher fatty acid, and

a pharmaceutically acceptable salt thereof,
for use as a radiosensitizer in combination with irradiation in an anti-tumor radiation treating method.

**8.** The compound according to claim 7, wherein the compound is selected from the group consisting of a compound represented by the following general formula (2):

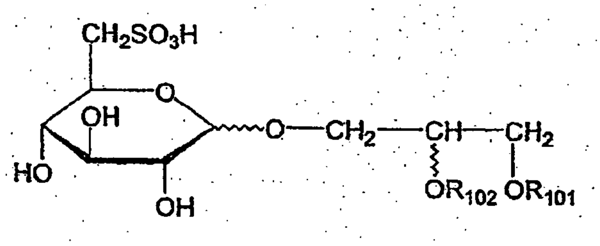

(2)

wherein $R_{101}$ represents an acyl residue of higher fatty acid, and $R_{102}$ represents a hydrogen atom or an acyl residue of higher fatty acid, and a pharmaceutically acceptable salt thereof.

**9.** The compound according to claim 8, wherein $R_{101}$ is R-CO- where R is an alkyl group having 13 to 25 carbon atoms, and $R_{102}$ is a hydrogen atom or R-CO- where R is an alkyl group having 13 to 25 carbon atoms in the general formula (2).

**10.** The compound according to claim 9, wherein $R_{101}$ is R-CO- where R is a straight alkyl group having an odd carbon number of 13 to 25 in the general formula (2).

**11.** The compound according to claim 9 or 10, wherein $R_{102}$ is a hydrogen atom in the general formula (2).

**12.** The compound according to claim 9 or 10, wherein $R_{102}$ is R-CO- where R is an alkyl group having 13 to 25 carbon atoms in the general formula (2).

**13.** A pharmaceutical composition which comprises, as an effective ingredient, at least one kind of compound selected from the group consisting of a compound represented by the following general formula (1):

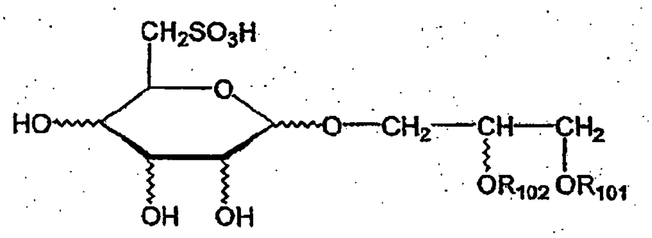

(1)

wherein $R_{101}$, represents an acyl residue of higher fatty acid, and $R_{102}$ represents a hydrogen atom or an acyl residue of higher fatty acid, and
a pharmaceutically acceptable salt thereof,
and a pharmaceutically acceptable excipient or diluent
for use as a radiosensitizer in combination with irradiation in an anti-tumor radiation treating method.

**14.** The pharmaceutical composition according to claim 13, wherein the compound is selected from the group consisting of a compound represented by the following general formula (2):

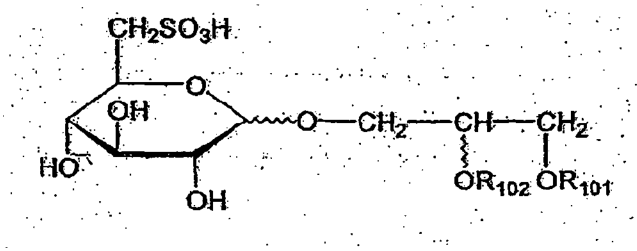

(2)

wherein $R_{101}$ represents an acyl residue of higher fatty acid, and $R_{102}$ represents a hydrogen atom or an acyl residue of higher fatty acid and a pharmaceutically acceptable salt thereof.

**15.** The pharmaceutical composition according to claim 14, wherein $R_{101}$ is R-CO- where R is an alkyl group having 13 to 25 carbon atoms and $R_{102}$ is a hydrogen atom or R-CO- where R is an alkyl group having 13 to 25 carbon atoms in the general formula (2).

**16.** The pharmaceutical composition according to claim 15, wherein $R_{101}$ is R-CO- where R is a straight alkyl group having an odd carbon number of 13 to 25 in the general formula (2).

**17.** The pharmaceutical composition according to claim 15 or 16, wherein $R_{102}$ is a hydrogen atom in the general formula (2).

**18.** The pharmaceutical composition according to claim 15 or 16, wherein $R_{102}$ is R-CO- where R is an alkyl group having 13 to 25 carbon atoms in the general formula (2).

**19.** A radiosensitizer which comprises, as an effective ingredient, at least one kind of compound selected from the group consisting of a compound represented by the following general formula (1):

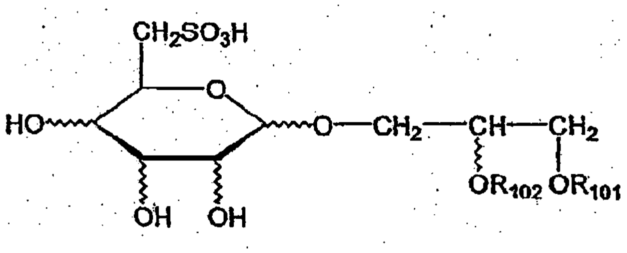

(1)

wherein $R_{101}$ represents an acyl residue of higher fatty acid, and $R_{102}$ represents a hydrogen atom or an acyl residue of higher fatty acid and
a pharmaceutically acceptable salt thereof,
for use in combination with irradiation in an anti-tumor radiation treating method.

**20.** The radiosensitizer according to claim 19, wherein the compound is selected from the group consisting of a compound represented by the following general formula (2):

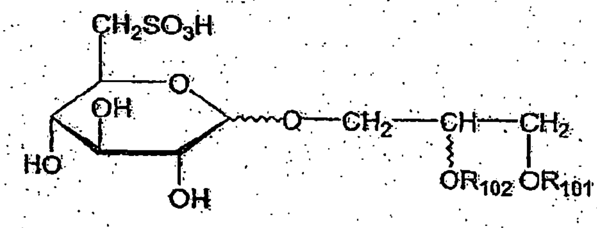

(2)

wherein $R_{101}$ represents an acyl residue of higher fatty acid, and $R_{102}$ represents a hydrogen atom or an acyl residue of higher fatty acid and a pharmaceutically acceptable salt thereof.

**21.** The radiosensitizer according to claim 20, wherein $R_{101}$ is R-CO- where R is an alkyl group having 13 to 25 carbon atoms, and $R_{102}$ is a hydrogen atom or R-CO- where R is an alkyl group having 13 to 25 carbon atoms in the general formula (2).

**22.** The radiosensitizer according to claim 21, wherein $R_{101}$ is R-CO- where R is a straight alkyl group having an odd carbon number of 13 to 25 in the general formula (2).

**23.** The radiosensitizer according to claim 21 or 22, wherein $R_{102}$ is a hydrogen atom in the general formula (2).

**24.** The radiosensitizer according to claim 21 or 22, wherein $R_{102}$ is R-CO-where R is an alkyl group having 13 to 25 carbon atoms in the general formula (2).

## Patentansprüche

**1.** Verwendung von mindestens einer Art von Verbindung, ausgewählt aus der Gruppe, welche aus einer Verbindung, die durch die folgende allgemeine Formel (1) repräsentiert wird:

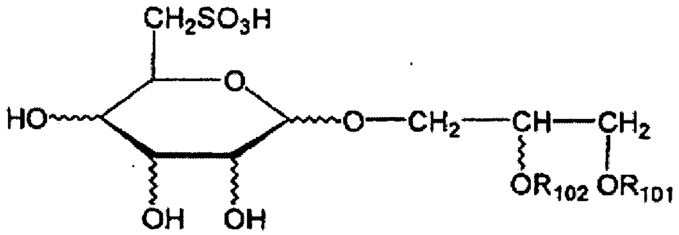

(1)

worin $R_{101}$ einen Acylrest einer höheren Fettsäure darstellt, und $R_{102}$ ein Wasserstoffatom oder einen Acylrest einer höheren Fettsäure darstellt, und
einem pharmazeutisch annehmbaren Salz davon besteht, zur Herstellung eines Radiosensibilisators zur Verwendung in Kombination mit Bestrahlung in einem Antitumor-Bestrahlungsbehandlungsverfahren.

**2.** verwendung nach Anspruch 1, wobei die Verbindung aus der Gruppe, welche aus einer Verbindung, die durch die folgende allgemeine Formel (2) repräsentiert wird:

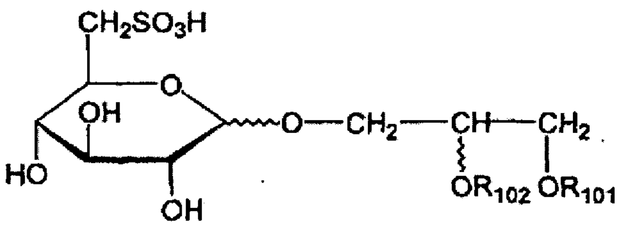

(2)

worin $R_{101}$ einen Acylrest einer höheren Fettsäure darstellt und $R_{102}$ ein Wasserstoffatom oder einen Acylrest einer höheren Fettsäure darstellt, und einem pharmazeutisch annehmbaren Salz davon besteht, ausgewählt ist.

**3.** Verwendung nach Anspruch 2, wobei in der allgemeinen Formel (2) $R_{101}$ R-CO-, worin R eine Alkylgruppe mit 13 bis 25 Kohlenstoffatomen ist, darstellt und $R_{102}$ ein Wasserstoffatom oder R-CO-, wrin R eine Alkylgruppe mit 13 bis 25 Kohlenstoffatomen ist, darstellt.

**4.** Verwendung nach Anspruch 3, wobei in der allgemeinen Formel (2) $R_{101}$ R-CO-, worin R eine geradkettige Alkylgruppe mit einer ungeraden Kohlenstoffanzahl von 13 bis 25 ist, darstellt.

**5.** Verwendung nach Anspruch 3 oder 4, wobei in der allgemeinen Formel (2) $R_{102}$ ein Wasserstoffatom darstellt.

6. Verwendung nach Anspruch 3 oder 4, wobei in der allgemeinen Formel (2) $R_{102}$ R-CO-, worin R eine Alkylgruppe mit 13 bis 25 Kohlenstoffatomen ist, darstellt.

7. Mindestens eine Art von Verbindung, ausgewählt aus der Gruppe, welche aus einer Verbindung, die durch die folgende allgemeine Formel (1) repräsentiert wird:

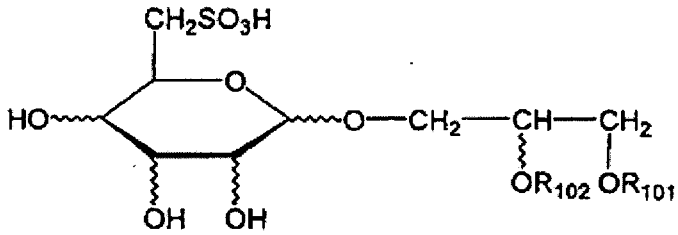

(1)

worin $R_{101}$ einen Acylrest einer höheren Fettsäure darstellt und $R_{102}$ ein Wasserstoffatom oder einen Acylrest einer höheren Fettsäure darstellt,
und einem pharmazeutisch annehmbaren Salz davon besteht,
zur Verwendung als Radiosensibilisator im Kombination mit Bestrahlung in einem Antitumor-Bestrahlungsbehandlungsverfahren.

8. Verbindung nach Anspruch 7, wobei die Verbindung aus der Gruppe ausgewählt ist, welche aus einer Verbindung, die durch die folgende allgemeine Formel (2) repräsentiert wird:

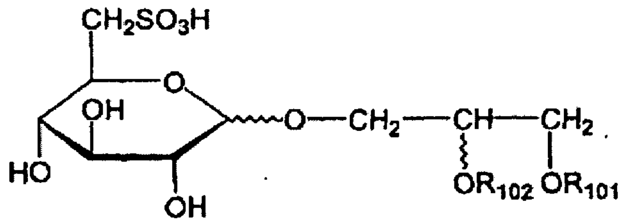

(2)

worin $R_{101}$ einen Acylrest einer höheren Fettsäure darstellt und $R_{102}$ ein Wasserstoffatom oder einen Acylrest einer höheren Fettsäure darstellt, und einem pharmazeutisch annehmbaren Salz davon besteht.

9. Verbindung nach Anspruch 8, wobei in der allgemeinen Formel (2) $R_{101}$ R-CO-, worin R eine Alkylgruppe mit 13 bis 25 Kohlenstoffatomen ist, darstellt und $R_{102}$ ein Wasserstoffatom oder R-CO-, worin R eine Alkylgruppe mit 13 bis 25 Kohlenstoffatomen ist, darstellt.

10. Verbindung nach Anspruch 9, wobei in der allgemeinen Formel (2) $R_{101}$ R-CO-, worin R eine geradkettige Alkylgruppe mit einer ungeraden Kohlenstoffanzahl von 13 bis 25 ist, darstellt.

11. Verbindung nach Anspruch 9 oder 10, wobei in der allgemeinen Formel (2) $R_{102}$ ein Wasserstoffatom darstellt.

12. Verbindung nach Anspruch 9 oder 10, wobei in der allgemeinen Formel (2) $R_{102}$ R-CO-, worin R eine Alkylgruppe mit 13 bis 25 Kohlenstoffatomen ist, darstellt.

13. Pharmazeutische Zusammensetzung, welche, als wirksamen Bestandteil, mindestens eine Art von Verbindung, ausgewählt aus der Gruppe, welche aus einer Verbindung, die durch die folgende allgemeine Formel (1) repräsentiert wird:

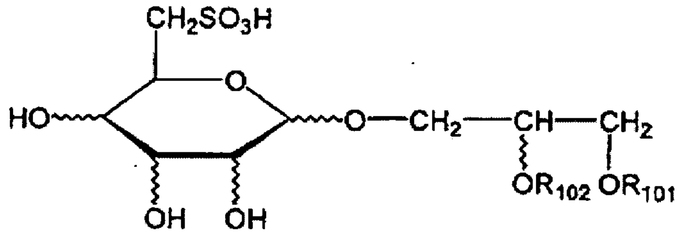

(1)

worin $R_{101}$ einen Acylrest einer höheren Fettsäure darstellt und $R_{102}$ ein Wasserstoffatom oder einen Acylrest einer höheren Fettsäure darstellt, und einem pharmazeutisch annehmbaren Salz davon besteht,
sowie einen pharmazeutisch annehmbaren Exzipienten oder ein pharmazeutisch annehmbares Verdünnungsmittel umfasst, zur Verwendung als Radiosensibilisator in Kombination mit Bestrahlung in einem Antitumor-Bestrahlungsbehandlungsverfahren.

**14.** Pharmazeutische Zusammensetzung nach Anspruch 13, wobei die Verbindung aus der Gruppe, welche aus einer Verbindung, die durch die folgende allgemeine Formel (2) repräsentiert wird:

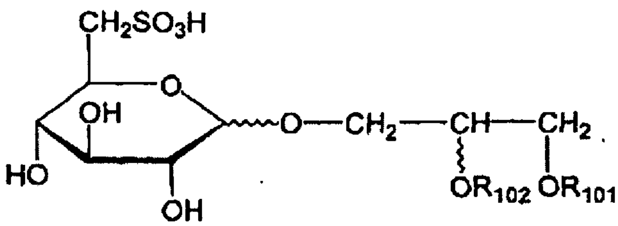

(2)

worin $R_{101}$ einen Acylrest einer höheren Fettsäure darstellt und $R_{102}$ ein Wasserstoffatom oder einen Acylrest einer höheren Fettsäure darstellt, und einem pharmazeutisch annehmbaren Salz davon besteht, ausgewählt ist.

**15.** Pharmazeutische Zusammensetzung nach Anspruch 14, wobei in der allgemeinen Formel (2) $R_{101}$ R-CO-, worin R eine Alkylgruppe mit 13 bis 25 Kohlenstoffatomen ist, darstellt und $R_{102}$ ein Wasserstoffatom oder R-CO-, worin R eine Alkylgruppe mit 13 bis 25 Kohlenstoffatomen ist, darstellt.

**16.** Pharmazeutische Zusammensetzung nach Anspruch 15, wobei in der allgemeinen Formel (2) $R_{101}$ R-CO-, worin R eine geradkettige Alkygruppe mit einer ungeraden Zahl von Kohlenstoffatomen von 13 bis 25 ist, darstellt.

**17.** Pharmazeutische Zusammensetzung nach Anspruch 15 oder 16, wobei in der allgemeinen Formel (2) $R_{102}$ ein Wasserstoffatom darstellt.

**18.** Pharmazeutische Zusammensetzung nach Anspruch 15 oder 16, wobei in der allgemeinen Formel (2) $R_{102}$ R-CO-, worin R eine Alkylgruppe mit 13 bis 25 Kohlenstoffatomen ist, darstellt.

**19.** Radiosensibilisator, umfassend als wirksamen Bestandteil mindestens eine Art von Verbindung, welche ausgewählt ist aus der Gruppe, welche aus einer Verbindung, die durch die folgende allgemeine Formel (1) repräsentiert wird:

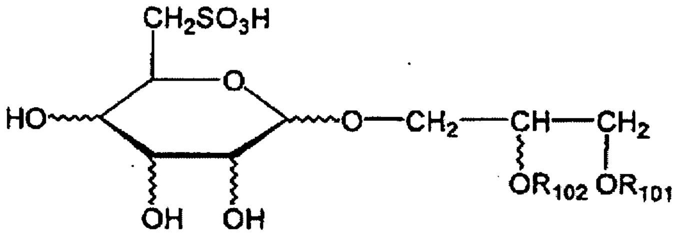

(1)

worin $R_{101}$ einen Acylrest einer höheren Fettsäure darstellt und $R_{102}$ ein Wasserstoffatom oder einen Acylrest einer höheren Fettsäure darstellt, und
einem pharmazeutisch annehmbaren Salz davon besteht, zur Verwendung in Kombination mit Bestrahlung in einem Anti-tumor-Bestrahlungsbehandlungsverfahren.

**20.** Radiosensibilisator nach Anspruch 19, wobei die Verbindung aus der Gruppe, welche aus einer Verbindung, die durch die folgende allgemeine Formel (2) repräsentiert wird:

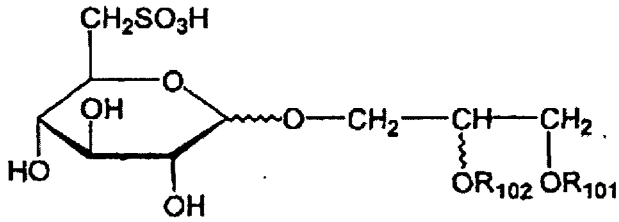

(2)

worin $R_{101}$ einen Acylrest einer höheren Fettsäure darstellt und $R_{102}$ ein Wasserstoffatom oder einen Acylrest einer höheren Fettsäure darstellt,
und einem pharmazeutisch annehmbaren Salz davon besteht, ausgewählt ist.

**21.** Radiosensibilisator nach Anspruch 20, wobei in der allgemeinen Formel (2) $R_{101}$ R-CO-, worin R eine Alkygruppe mit 13 bis 25 Kohlenstoffatomen ist, darstellt und $R_{102}$ ein Wasserstoffatom oder R-CO-, worin R eine Alkylgruppe mit 13 bis 25 Kohlenstoffatomen ist, darstellt.

**22.** Radiosensibilisator nach Anspruch 21, wobei in der allgemeinen Formel (2) $R_{101}$ R-CO-, worin R eine geradkettige Alkygruppe mit einer ungeraden Zahl von Kohlenstoffatomen von 13 bis 25 ist, darstellt.

**23.** Radiosensibilisator nach Anspruch 21 oder 22, wobei in der allgemeinen Formel (2) $R_{102}$ ein Wasserstoffatom darstellt.

**24.** Radiosensibilisator nach Anspruch 21 oder 22, wobei in der allgemeinen Formel (2) $R_{102}$ R-CO-, worin R eine Alkylgruppe mit 13 bis 25 Kohlenstoffatomen ist, darstellt.

## Revendications

**1.** Utilisation d'au moins un type de composé choisi parmi le groupe consistant en un composé représenté par la formule générale (1) suivante :

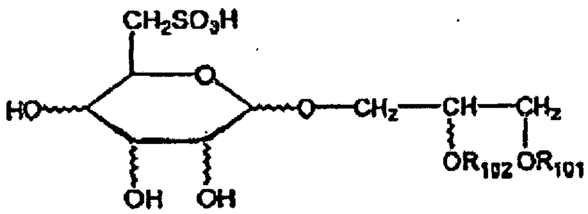

(1)

dans laquelle $R_{101}$ représente un résidu acyle d'acide gras supérieur, et $R_{102}$ représente un atome d'hydrogène ou un résidu acyle d'acide gras supérieur, et
un sel pharmaceutiquement acceptable de celui-ci,
pour la préparation d'un radiosensibilisateur destiné à une utilisation en combinaison avec une irradiation dans un procédé de traitement antitumoral par radiations.

**2.** Utilisation selon la revendication 1, dans laquelle le composé est choisi parmi le groupe consistant en un composé représenté par la formule générale (2) suivante :

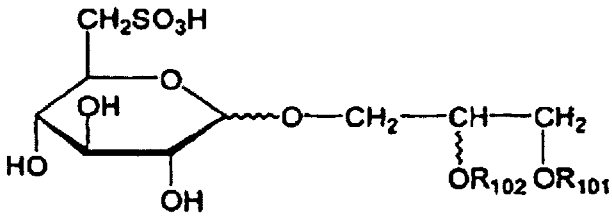

(2)

dans laquelle $R_{101}$ représente un résidu acyle d'acide gras supérieur, et $R_{102}$ représente un atome d'hydrogène ou un résidu acyle d'acide gras supérieur, et un sel pharmaceutiquement acceptable de celui-ci.

**3.** Utilisation selon la revendication 2, dans laquelle $R_{101}$ est R-CO-, où R est un groupe alkyle ayant 13 à 25 atomes de carbone, et $R_{102}$ est un atome d'hydrogène ou R-CO-, où R est un groupe alkyle ayant 13 à 25 atomes de carbone dans la formule générale (2).

**4.** Utilisation selon la revendication 3, dans laquelle $R_{101}$ est R-CO-, où R est un groupe alkyle linéaire ayant un nombre impair de carbones de 13 à 25 dans la formule générale (2).

**5.** Utilisation selon la revendication 3 ou 4, dans laquelle $R_{102}$ est un atome d'hydrogène dans la formule générale (2).

**6.** Utilisation selon la revendication 3 ou 4, dans laquelle $R_{102}$ est R-CO-, où R est un groupe alkyle ayant 13 à 25 atomes de carbone dans la formule générale (2).

**7.** Au moins un type de composé choisi parmi le groupe consistant en un composé représenté par la formule générale (1) suivante :

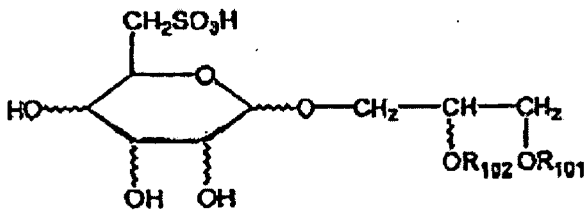

(1)

dans laquelle $R_{101}$ représente un résidu acyle d'acide gras supérieur, et $R_{102}$ représente un atome d'hydrogène ou un résidu acyle d'acide gras supérieur, et
un sel pharmaceutiquement acceptable de celui-ci,
destiné à une utilisation comme un radiosensibilisateur en combinaison avec une irradiation dans un procédé de traitement antitumoral par radiations.

**8.** Composé selon la revendication 7, dans lequel le composé est choisi parmi le groupe consistant en un composé représenté par la formule générale (2) suivante :

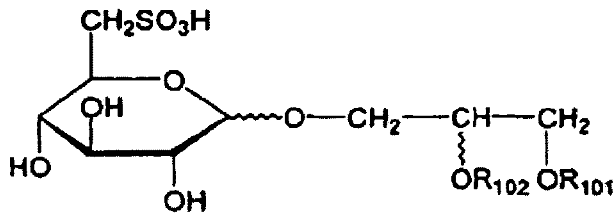

(2)

dans laquelle $R_{101}$ représente un résidu acyle d'acide gras supérieur, et $R_{102}$ représente un atome d'hydrogène ou un résidu acyle d'acide gras supérieur, et un sel pharmaceutiquement acceptable de celui-ci.

**9.** Composé selon la revendication 8, dans lequel $R_{101}$ est R-CO-, où R est un groupe alkyle ayant 13 à 25 atomes de carbone, et $R_{102}$ est un atome d'hydrogène ou R-CO-, où R est un groupe alkyle ayant 13 à 25 atomes de carbone dans la formule générale (2).

**10.** Composé selon la revendication 9, dans lequel $R_{101}$ est R-CO-, où R est un groupe alkyle linéaire ayant un nombre impair de carbones de 13 à 25 dans la formule générale (2).

**11.** Composé selon la revendication 9 ou 10, dans lequel $R_{102}$ est un atome d'hydrogène dans la formule générale (2).

**12.** Composé selon la revendication 9 ou 10, dans lequel $R_{102}$ est R-CO-, où R est un groupe alkyle ayant 13 à 25 atomes de carbone dans la formule générale (2).

**13.** Composition pharmaceutique qui comprend, comme un ingrédient efficace, au moins un type de composé choisi parmi le groupe consistant en un composé représenté par la formule générale (1) suivante :

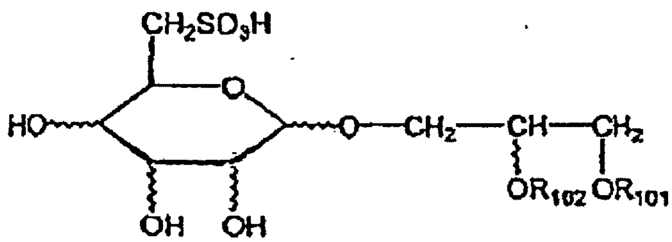

(1)

dans laquelle $R_{101}$ représente un résidu acyle d'acide gras supérieur, et $R_{102}$ représente un atome d'hydrogène ou un résidu acyle d'acide gras supérieur, et
un sel pharmaceutiquement acceptable de celui-ci, et un excipient ou un diluant pharmaceutiquement acceptable, destinée à une utilisation comme un radiosensibilisateur en combinaison avec une irradiation dans un procédé de traitement antitumoral par radiations.

**14.** Composition pharmaceutique selon la revendication 13, dans laquelle le composé est choisi parmi le groupe consistant en un composé représenté par la formule générale (2) suivante :

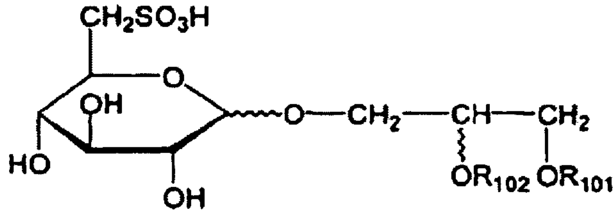

(2)

dans laquelle $R_{101}$ représente un résidu acyle d'acide gras supérieur, et $R_{102}$ représente un atome d'hydrogène ou un résidu acyle d'acide gras supérieur, et un sel pharmaceutiquement acceptable de celui-ci.

**15.** Composition pharmaceutique selon la revendication 14, dans laquelle $R_{101}$ est R-CO-, où R est un groupe alkyle ayant 13 à 25 atomes de carbone, et $R_{102}$ est un atome d'hydrogène ou R-CO-, où R est un groupe alkyle ayant 13 à 25 atomes de carbone dans la formule générale (2).

**16.** Composition pharmaceutique selon la revendication 15, dans laquelle $R_{101}$ est R-CO-, où R est un groupe alkyle linéaire ayant un nombre impair de carbones de 13 à 25 dans la formule générale (2).

**17.** Composition pharmaceutique selon la revendication 15 ou 16, dans laquelle $R_{102}$ est un atome d'hydrogène dans la formule générale (2).

**18.** Composition pharmaceutique selon la revendication 15 ou 16, dans laquelle $R_{102}$ est R-CO-, où R est un groupe alkyle ayant 13 à 25 atomes de carbone dans la formule générale (2).

**19.** Radiosensibilisateur qui comprend, comme un ingrédient efficace, au moins un type de composé choisi parmi le groupe consistant en
un composé représenté par la formule générale (1) suivante :

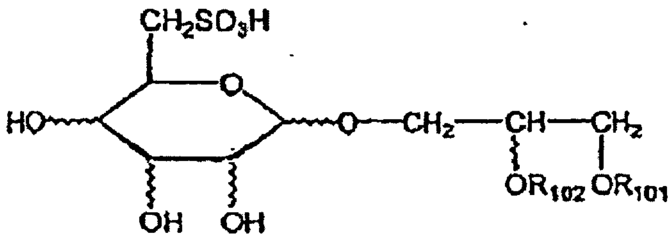

(1)

dans laquelle $R_{101}$ représente un résidu acyle d'acide gras supérieur, et $R_{102}$ représente un atome d'hydrogène ou un résidu acyle d'acide gras supérieur, et
un sel pharmaceutiquement acceptable de celui-ci, destiné à une utilisation en combinaison avec une irradiation dans un procédé de traitement antitumoral par radiations.

**20.** Radiosensibilisateur selon la revendication 19, dans lequel le composé est choisi parmi le groupe consistant en un composé représenté par la formule générale (2) suivante :

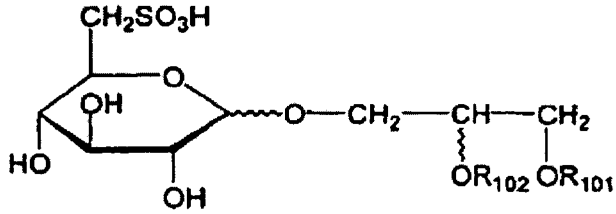

(2)

dans laquelle $R_{101}$ représente un résidu acyle d'acide gras supérieur, et $R_{102}$ représente un atome d'hydrogène ou un résidu acyle d'acide gras supérieur, et un sel pharmaceutiquement acceptable de celui-ci.

**21.** Radiosensibilisateur selon la revendication 20, dans lequel $R_{101}$ est R-CO-, où R est un groupe alkyle ayant 13 à 25 atomes de carbone, et $R_{102}$ est un atome d'hydrogène ou R-CO-, où R est un groupe alkyle ayant 13 à 25 atomes de carbone dans la formule générale (2).

**22.** Radiosensibilisateur selon la revendication 21, dans lequel $R_{101}$ est R-CO-, où R est un groupe alkyle linéaire ayant un nombre impair de carbones de 13 à 25 dans la formule générale (2).

**23.** Radiosensibilisateur selon la revendication 21 ou 22, dans lequel $R_{102}$ est un atome d'hydrogène dans la formule générale (2).

**24.** Radiosensibilisateur selon la revendication 21 ou 22, dans lequel $R_{102}$ est R-CO-, où R est un groupe alkyle ayant 13 à 25 atomes de carbone dans la formule générale (2).

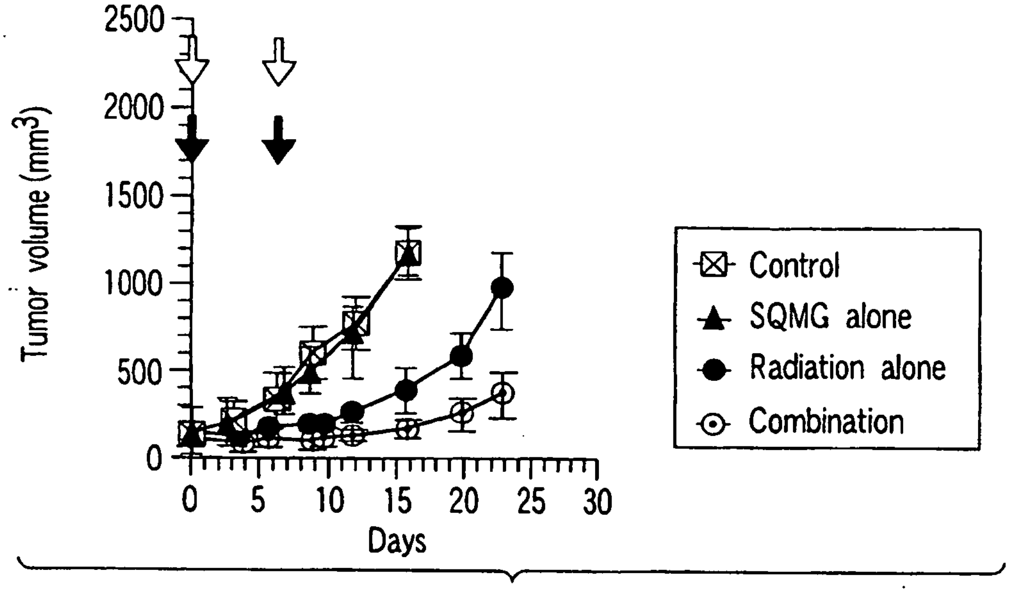
Tumor volume (mm$^3$)
2500
2000
1500
1000
500
0
0 5 10 15 20 25 30
Days
Control
SQMG alone
Radiation alone
Combination

FIG. 1

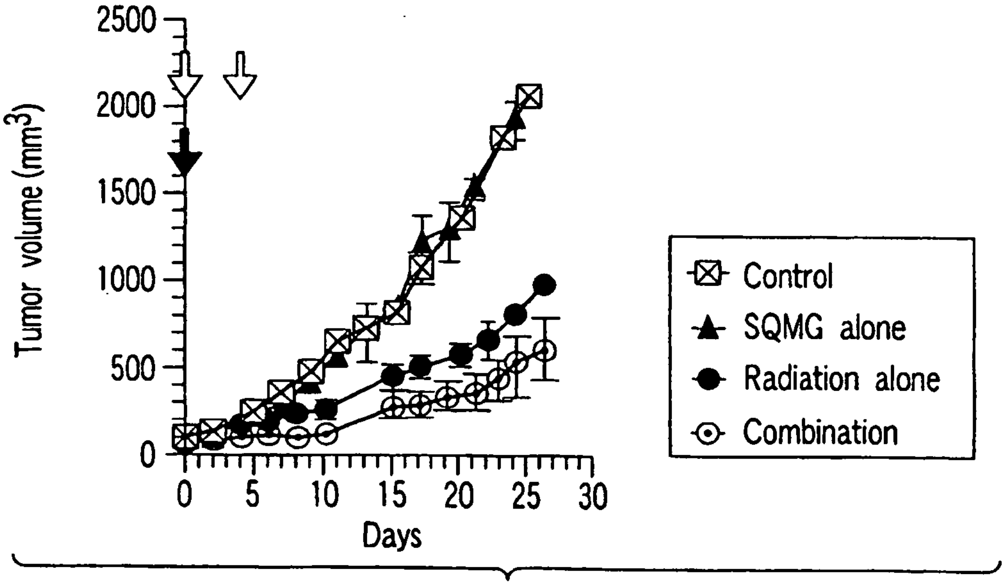
Tumor volume (mm$^3$)
2500
2000
1500
1000
500
0
0 5 10 15 20 25 30
Days
Control
SQMG alone
Radiation alone
Combination

FIG. 2

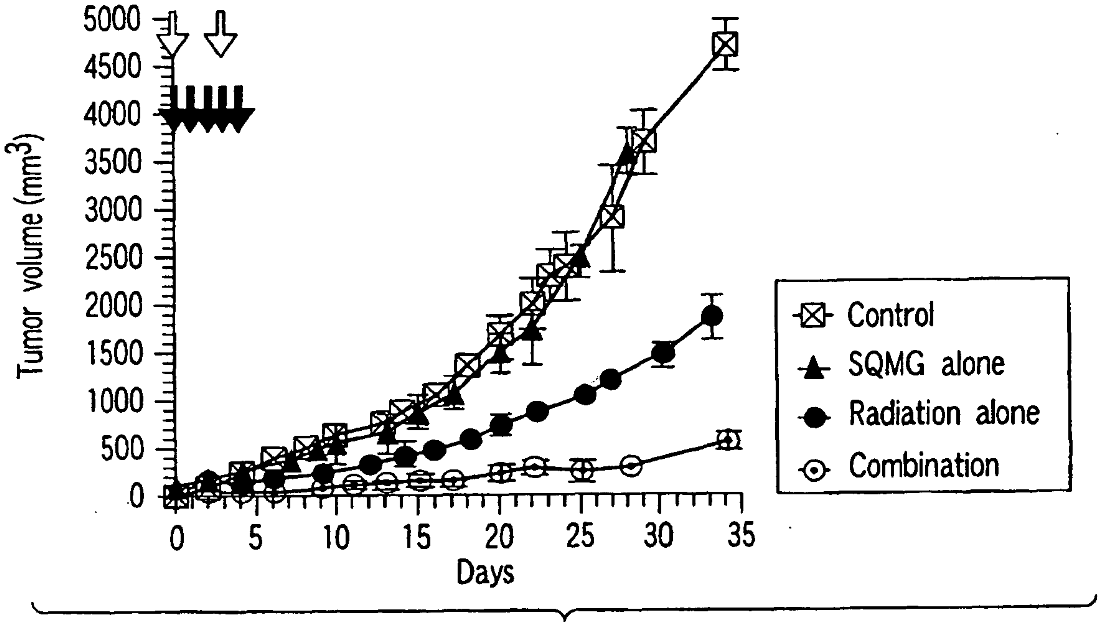
5000
4500
4000
3500
3000
2500
2000
1500
1000
500
0
Tumor volume (mm3)
0
5
10
15
20
25
30
35
Days
Control
SQMG alone
Radiation alone
Combination

FIG. 3

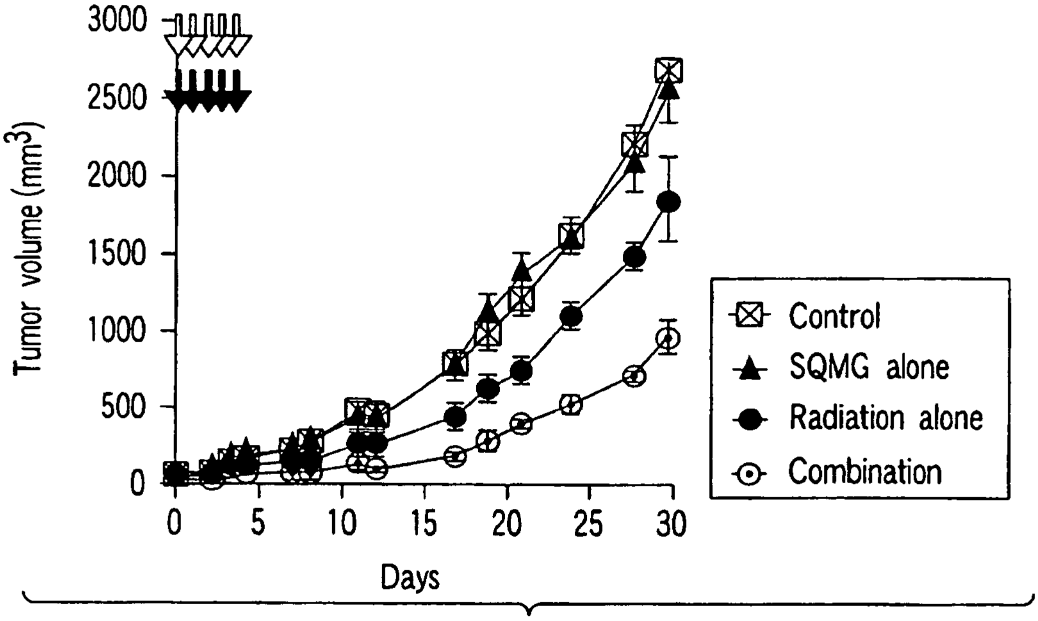
3000
2500
2000
1500
1000
500
0
Tumor volume (mm3)
0
5
10
15
20
25
30
Days
Control
SQMG alone
Radiation alone
Combination

FIG. 4

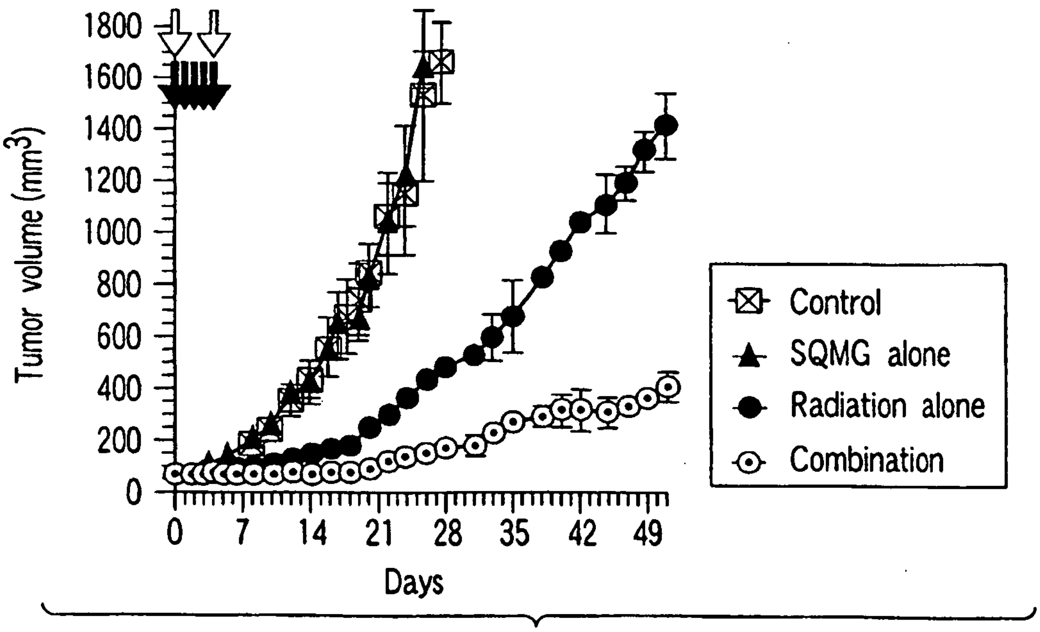
1800
1600
1400
1200
1000
800
600
400
200
0
Tumor volume (mm3)
0
7
14
21
28
35
42
49
Days
Control
SQMG alone
Radiation alone
Combination

FIG. 5

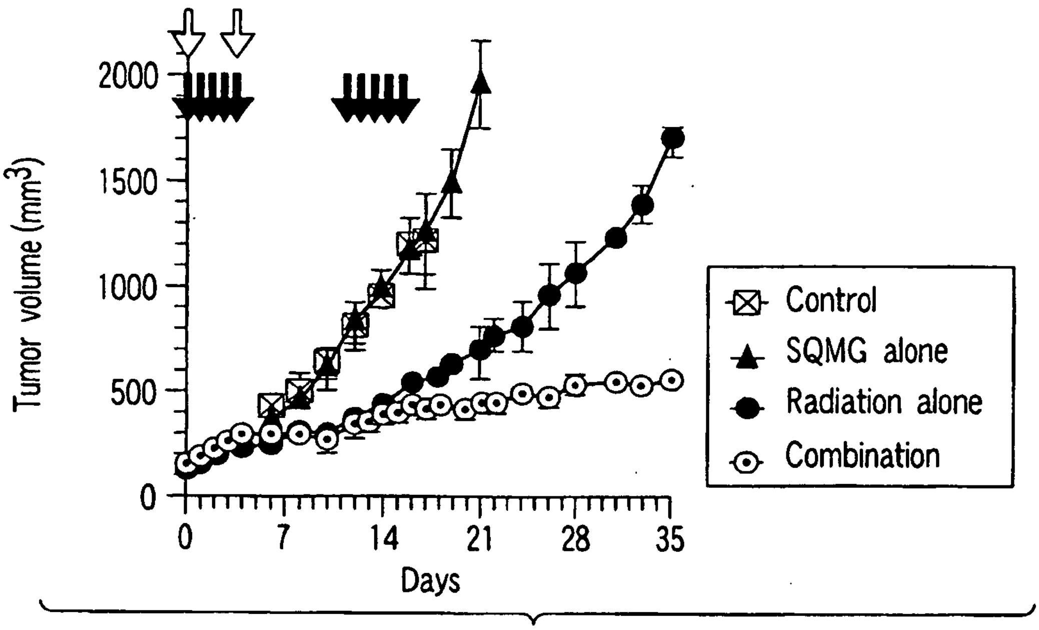
2000
1500
1000
500
0
Tumor volume (mm3)
0
7
14
21
28
35
Days
Control
SQMG alone
Radiation alone
Combination

FIG. 6

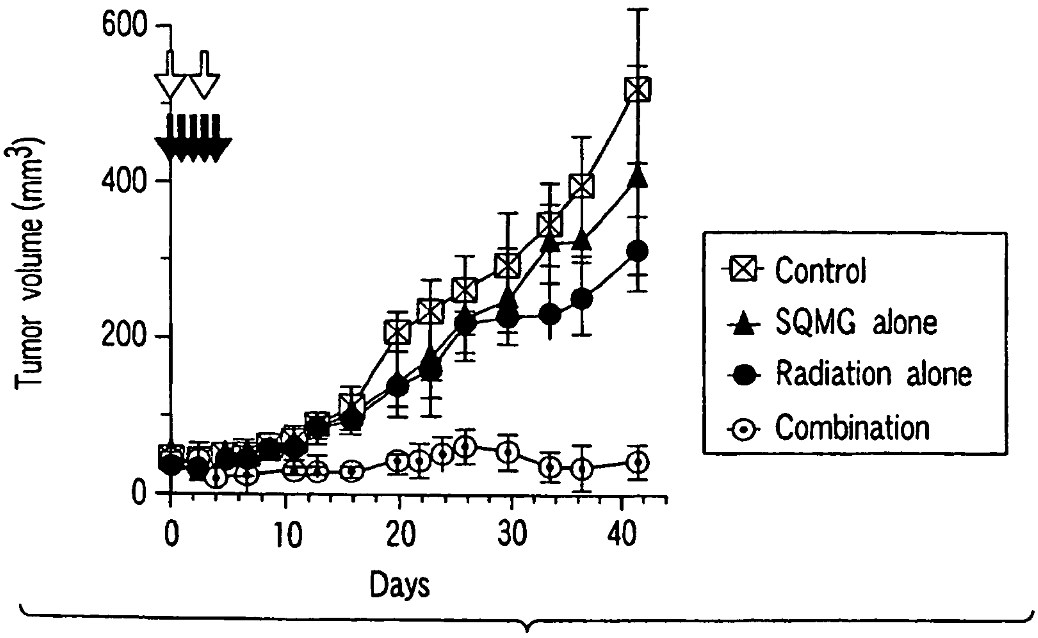
600
400
200
0
Tumor volume (mm3)
0
10
20
30
40
Days
Control
SQMG alone
Radiation alone
Combination

FIG. 7

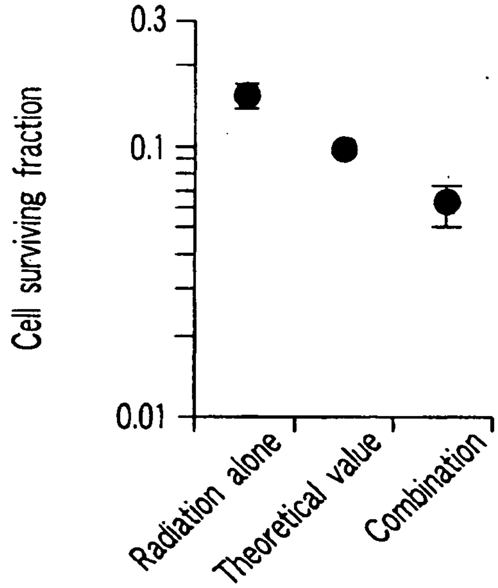
0.3
0.1
0.01
Cell surviving fraction
Radiation alone
Theoretical value
Combination

FIG. 8

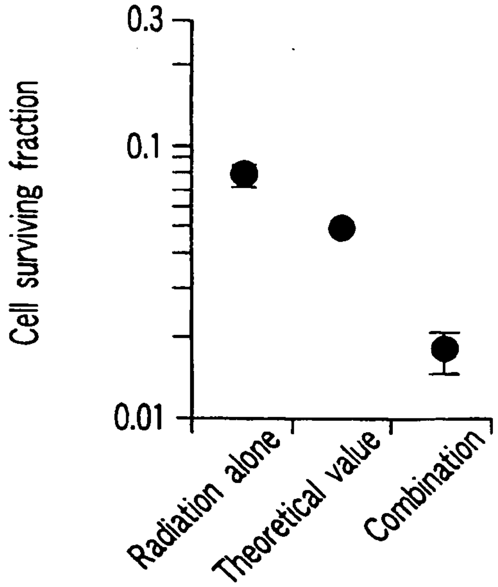
0.3
0.1
0.01
Cell surviving fraction
Radiation alone
Theoretical value
Combination

FIG. 9

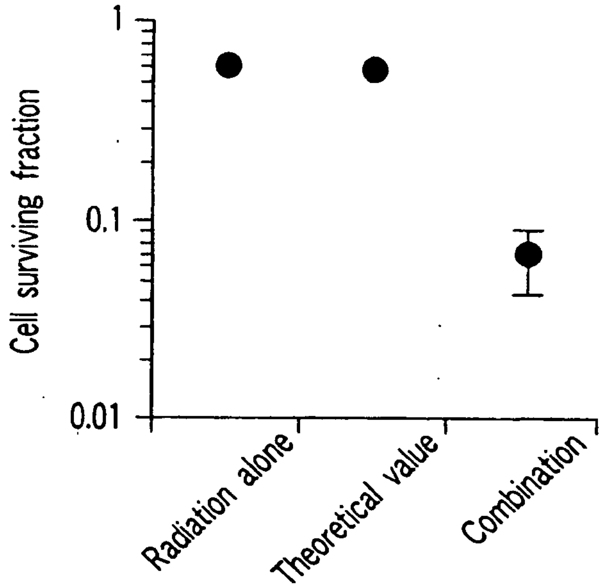
1
0.1
0.01
Cell surviving fraction
Radiation alone
Theoretical value
Combination

F I G. 10

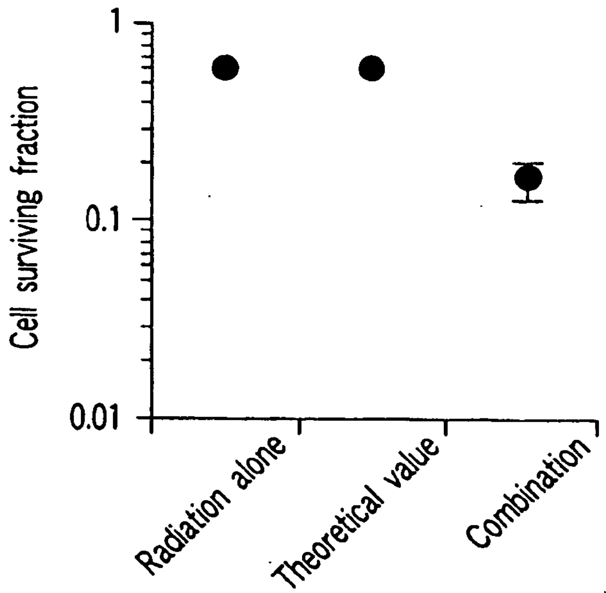
1
0.1
0.01
Cell surviving fraction
Radiation alone
Theoretical value
Combination

F I G. 11

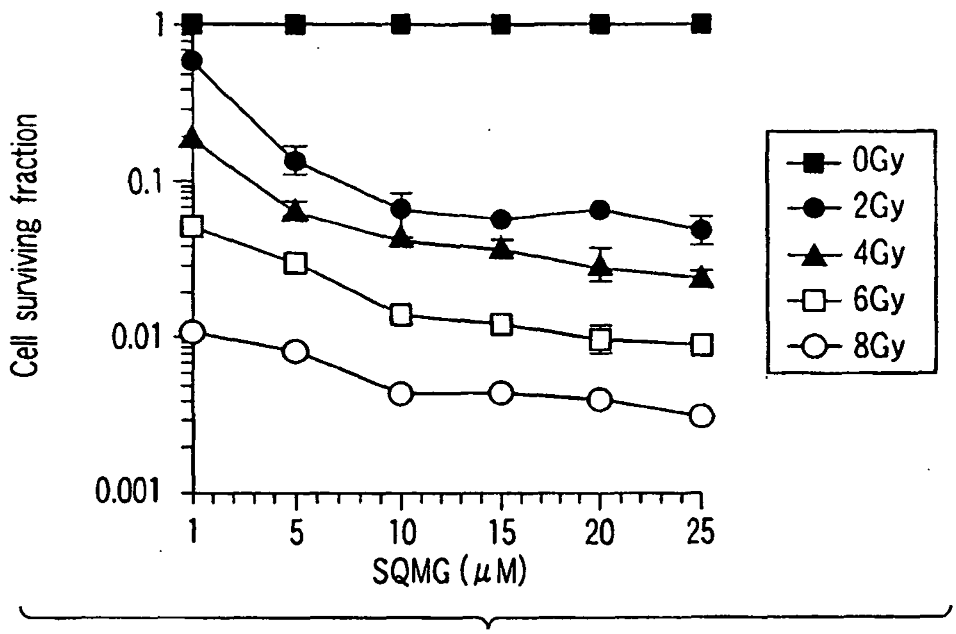
1
0.1
0.01
0.001
Cell surviving fraction
1
5
10
15
20
25
SQMG (μM)
0Gy
2Gy
4Gy
6Gy
8Gy

FIG. 12

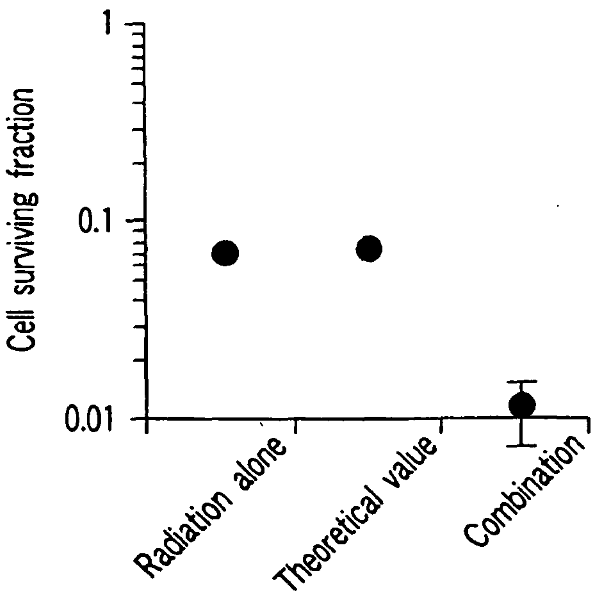
1
0.1
0.01
Cell surviving fraction
Radiation alone
Theoretical value
Combination

FIG. 13

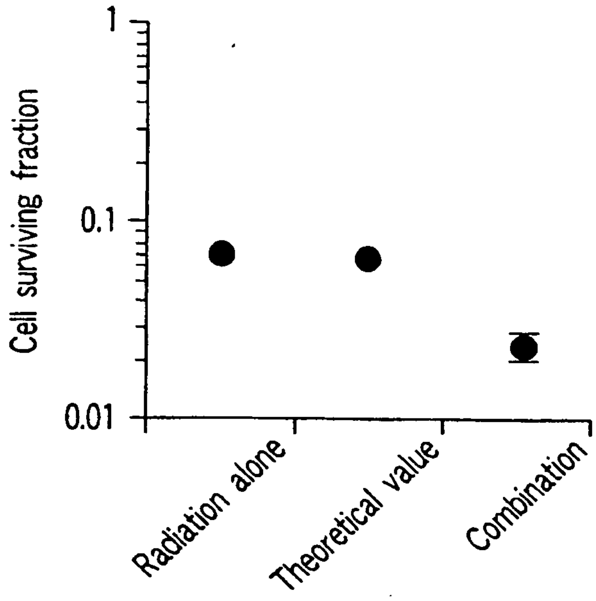
1
0.1
0.01
Cell surviving fraction
Radiation alone
Theoretical value
Combination

F I G. 14

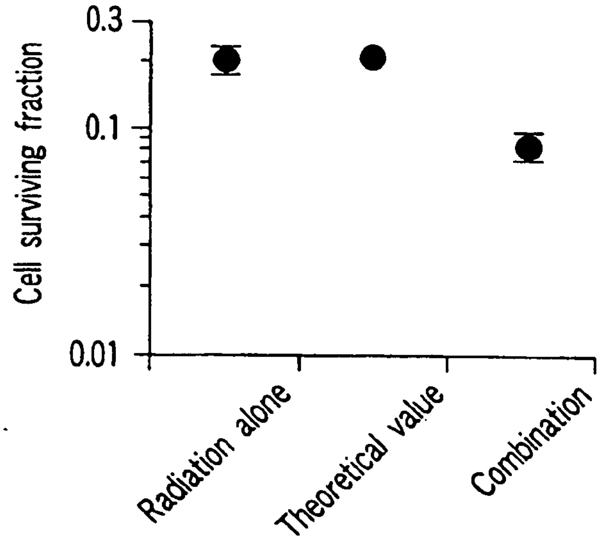
0.3
0.1
0.01
Cell surviving fraction
Radiation alone
Theoretical value
Combination

F I G. 15

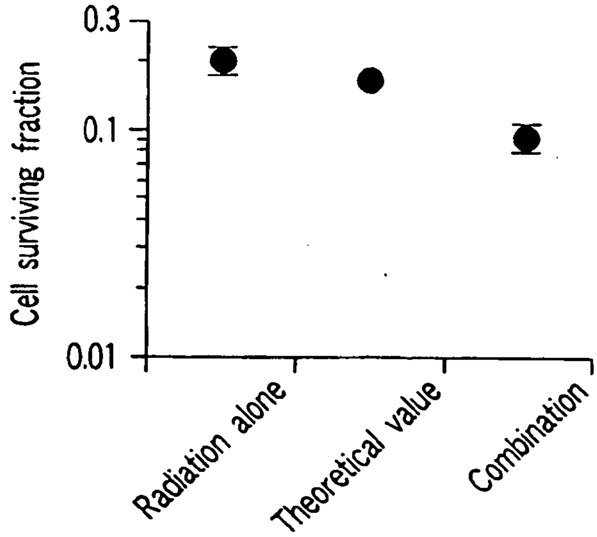
0.3
0.1
0.01
Cell surviving fraction
Radiation alone
Theoretical value
Combination

FIG. 16

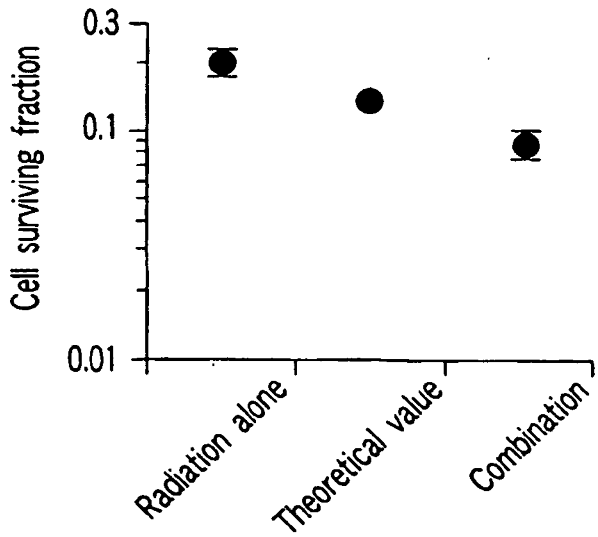
0.3
0.1
0.01
Cell surviving fraction
Radiation alone
Theoretical value
Combination

FIG. 17

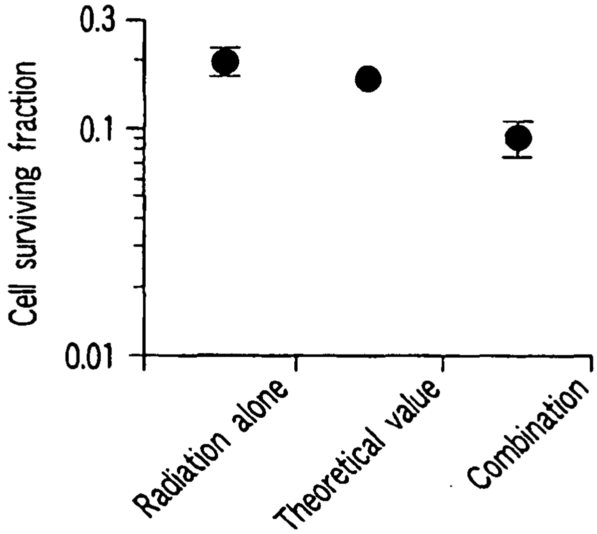
0.3
0.1
0.01
Cell surviving fraction
Radiation alone
Theoretical value
Combination

FIG. 18

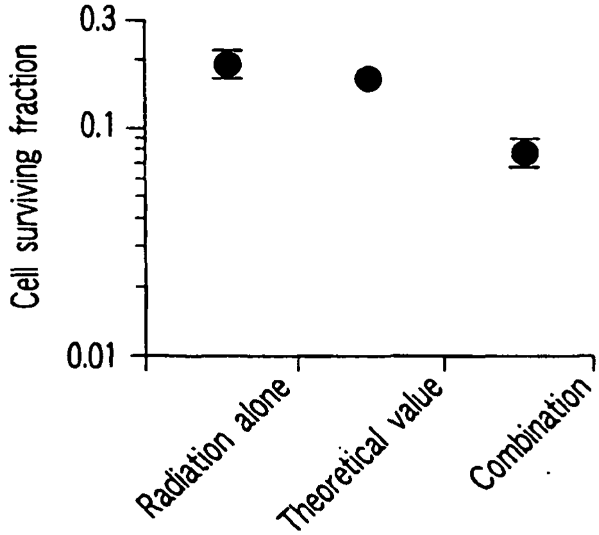
0.3
0.1
0.01
Cell surviving fraction
Radiation alone
Theoretical value
Combination

FIG. 19

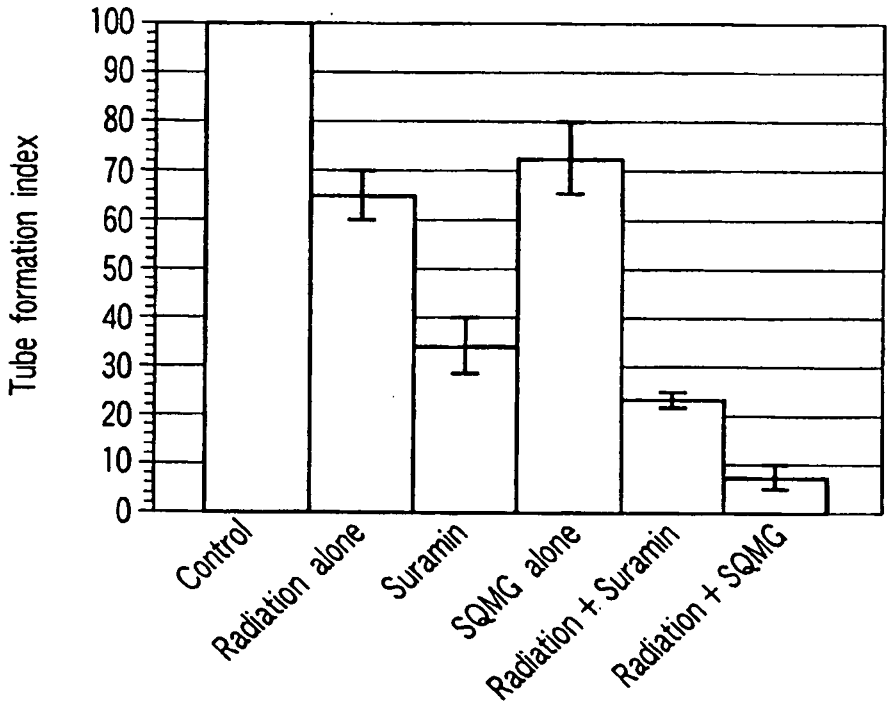
Tube formation index
100
90
80
70
60
50
40
30
20
10
0
Control
Radiation alone
Suramin
SQMG alone
Radiation + Suramin
Radiation + SQMG

FIG. 20

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0051622 A1
- WO 0052021 A1
- WO 9634872 A
- JP 57067518 A
- JP 57035516 A
- JP 2000212087 A
- WO 0052020 A
- WO 0052021 A
- WO 0051622 A


### Non-patent literature cited in the description


- **Eric J. Hall et al.** Radiobiology for the Radiologist. J.B. Lippincott Company
- **Ohta et al.** *Biol. Pharm. Bull.,* 1999, vol. 22 (2), 111-116
- *Int., J. Radiation Oncology Bio. Phys.,* 2003, vol. 55 (3), 713-723